(19) Europäisches Patentamt
European Patent Office
Office européen des brevets

(11) EP 4 670 706 A1

(12) EUROPEAN PATENT APPLICATION
published in accordance with Art. 153(4) EPC

(43) Date of publication:
31.12.2025 Bulletin 2026/01

(21) Application number: 24760387.1

(22) Date of filing: 21.02.2024

(51) International Patent Classification (IPC):
*A61K 8/27* (2006.01) *A61K 8/06* (2006.01)
*A61K 8/29* (2006.01) *A61K 8/34* (2006.01)
*A61K 8/44* (2006.01) *A61K 8/73* (2006.01)
*A61Q 17/04* (2006.01)

(52) Cooperative Patent Classification (CPC):
**A61K 8/06; A61K 8/27; A61K 8/29; A61K 8/34; A61K 8/44; A61K 8/73; A61Q 17/04**

(86) International application number:
PCT/JP2024/006148

(87) International publication number:
WO 2024/177089 (29.08.2024 Gazette 2024/35)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: 21.02.2023 JP 2023025333

(71) Applicant: **Kao Corporation**
**Chuo-ku**
**Tokyo 103-8210 (JP)**

(72) Inventors:
- **CHEN Shi**
  **Tokyo 131-8501 (JP)**
- **ISHITA Mio**
  **Tokyo 131-8501 (JP)**

(74) Representative: **Hoffmann Eitle**
**Patent- und Rechtsanwälte PartmbB**
**Arabellastraße 30**
**81925 München (DE)**

# (54) WATER-IN-OIL EMULSION COSMETIC

(57) The present invention relates to a water-in-oil emulsified cosmetic containing the following components (A), (B), and (C): (A) an UV scattering agent having a hydrophobicity degree of 73% or less in accordance with methanol titration; (B) an amphiphilic substance which is solid at 25°C; and (C) an aqueous-phase component, the component (A) containing dimethicone-treated zinc oxide microparticles (Al), in which a mass ratio of a content of the component (Al) to a content of the component (A) [component (A1)/component (A)] being 0.38 or more, the component (C) having a surface tension of less than 0.0728 N/m, and a content of the component (C) being 16 mass% or more.



Processed by Luminess, 75001 PARIS (FR)

## Description

### Field of the Invention

The present invention relates to a water-in-oil emulsified cosmetic.

### Background of the Invention

In recent years, as the importance of protection from UV rays in daily life increases, the demand for sunscreen cosmetics is also expanding. In response to such market demands, water-in-oil emulsified sunscreen cosmetics are being developed.

For these water-in-oil emulsified cosmetics, UV scattering agents, UV absorbers, and other UV protection components are used in order to enhance their UV protection effects. Particularly as UV scattering agents, metal oxide powders, such as zinc oxide and titanium oxide, are used.

For example, for the purpose of providing a water-in-oil composition with an oil phase blended with a hydrophobized powder, in which the powder is unlikely to aggregate or settle, WO 2021/044975 (Patent Literature 1) describes a water-in-oil composition containing an oil phase having a mass ratio of silicone oil/oil phase of 0.65 or more, a silicone-treated powder dispersed in the oil phase and treated with a silicone-based surface treatment agent, a polyglycerin-modified silicone dispersant which disperses the silicone-treated powder in the oil phase, an aqueous phase dispersed in the oil phase, and an emulsifier which disperses the aqueous phase in the oil phase, in which the mass ratio of the silicone-treated powder to all the powders is 0.7 or more.

For the purpose of providing a titanium oxide powder which can achieve a natural skin finish and a shading effect when the powder is applied to the skin, WO 2019/225491 (Patent Literature 2) describes a coated titanium oxide powder having a silicon oxide or hydroxide layer, an aluminum oxide or hydroxide layer, and an iron oxide or hydroxide layer, as well as a cosmetic or a skin external preparation, and the like containing the coated titanium oxide powder.

### Summary of the Invention

The present invention relates to a water-in-oil emulsified cosmetic comprising the following components (A), (B), and (C):

(A) an UV scattering agent having a hydrophobicity degree of 73% or less in accordance with methanol titration;
(B) an amphiphilic substance which is solid at 25°C; and
(C) an aqueous-phase component (provided that the component (B) is excluded),

the component (A) comprising dimethicone-treated zinc oxide microparticles (A1), in which a mass ratio of a content of the component (A1) to a content of the component (A) [component (A1)/component (A)] being 0.38 or more,
the component (C) having a surface tension of less than 0.0728 N/m, and
a content of the component (C) being 16 mass% or more.

### Detailed Description of the Invention

However, it found that the techniques of Patent Literatures 1 and 2 cannot provide sufficient UV protection effects, and that when wearing a mask as a measure against hay fever and infectious diseases, the friction between the mask and the cosmetic coating film formed on the skin surface impairs the UV protection effect. Furthermore, in recent years, in order to reduce the influence on the environment, there is a demand for cosmetics which can form cosmetic coating films having excellent water resistance to successfully prevent UV protection components, such as UV scattering agents and UV absorbers, from leaking into water, even if the cosmetic coating film on the skin surface gets wet at the beach or pool in summer leisure activities.

The present invention relates to a water-in-oil emulsified cosmetic which can form a cosmetic coating film having an excellent UV protection effect, excellent wear resistance, and excellent water resistance to be able to effectively prevent UV protection components from leaking into water.

The present inventors found that when an UV scattering agent, the UV scattering agent being contains dimethicone-treated zinc oxide microparticles, whose hydrophobicity degree in accordance with methanol titration is equal to or less than a predetermined value, an amphiphilic substance which is solid at 25°C, and an aqueous-phase component are contained, and when the mass ratio of the content of the dimethicone-treated zinc oxide microparticles to the content of the UV scattering agent is equal to or greater than a predetermined value, the surface tension of the aqueous-phase component is less than a predetermined value, and the content of the aqueous-phase component is equal

to or greater than a predetermined value, it is possible to provide a water-in-oil emulsified cosmetic which can form a cosmetic coating film having an excellent UV protection effect, excellent wear resistance, and excellent water resistance to successfully prevent UV protection components from leaking into water.

That is, the present invention relates to a water-in-oil emulsified cosmetic comprising the following components (A), (B), and (C):

(A) an UV scattering agent having a hydrophobicity degree of 73% or less in accordance with methanol titration;
(B) an amphiphilic substance which is solid at 25°C; and
(C) an aqueous-phase component (provided that the component (B) is excluded),

the component (A) comprising dimethicone-treated zinc oxide microparticles (A1), in which a mass ratio of a content of the component (A1) to a content of the component (A) [component (A1)/component (A)] being 0.38 or more,
the component (C) having a surface tension of less than 0.0728 N/m, and
a content of the component (C) being 16 mass% or more.

The present invention can provide a water-in-oil emulsified cosmetic which can form a cosmetic coating film having an excellent UV protection effect, excellent wear resistance, and excellent water resistance to successfully prevent UV protection components from leaking into water.

[Water-in-oil emulsified cosmetic]

The water-in-oil emulsified cosmetic of the present invention (hereinafter also referred to as "the emulsified cosmetic") contains the following components (A), (B), and (C):

(A) an UV scattering agent having a hydrophobicity degree of 73% or less in accordance with methanol titration;
(B) an amphiphilic substance which is solid at 25°C; and
(C) an aqueous-phase component (provided that the component (B) is excluded),

the component (A) containing dimethicone-treated zinc oxide microparticles (A1), in which a mass ratio of a content of the component (A1) to a content of the component (A) [component (A1)/component (A)] being 0.38 or more,
the component (C) having a surface tension of less than 0.0728 N/m, and
a content of the component (C) being 16 mass% or more.

In the present invention, the phrase "comprising component X" or "containing component X" also means "being blended with component X."

In the present invention, the "aqueous-phase component" refers to water and a water-soluble component (provided that the component (B) is excluded). The "water-soluble component" as mentioned herein refers to a component which has a uniform appearance when visually observed in a mixture obtained by mixing 100 parts by mass of water and 3 parts by mass of the component at 25°C. The phrase that "the mixture has a uniform appearance" as mentioned herein means that no separation is visually observed, and that the mixture is transparent or opaque.

In the present invention, the "hydrophobicity degree (%) in accordance with methanol titration" (hereinafter also referred to simply as "hydrophobicity") is determined as follows. Specifically, 50 mL of ion exchange water and 0.2 g of an UV scattering agent are placed in a beaker, and methanol is added dropwise from a burette while the mixture in the beaker is stirred with a magnetic stirrer. Due to the increase in the concentration of methanol in the beaker, the UV scattering agent settles, and the mixture in the beaker becomes cloudy to reduce transmittance. At this point, the mass fraction of methanol in the water/methanol mixed solution at which the transmittance of the mixture after dropping methanol is 50% of the transmittance of the mixture before dropping methanol is regarded as hydrophobicity (%).

In the present invention, the term "solid" refers to a state which does not show fluidity and maintains a certain shape in an environment of 25°C under 1 atmosphere. In other words, this term refers to a state where the temperature is below the melting point (for an amorphous substance which does not have a melting point, a state where the temperature is below the softening point). The term "liquid" refers to a state which has fluidity in an environment of 25°C under 1 atmosphere, that is, a state where the temperature is equal to or higher than the melting point (for an amorphous substance which does not have a melting point, a state where the temperature is equal to or higher than the softening point).

In the present specification, a cosmetic coating film is formed by applying the water-in-oil emulsified cosmetic, and the UV protection effect of the cosmetic coating film after rubbing the cosmetic coating film is also referred to as "wear resistance." The effect of preventing UV protection components from leaking into water resulting from the improved water resistance of the cosmetic coating film formed by applying the water-in-oil emulsified cosmetic is also referred to as "water resistance."

The emulsified cosmetic of the present invention can form a cosmetic coating film which has an excellent UV protection effect, excellent wear resistance, and excellent water resistance to be able to effectively prevent UV protection components from leaking into water. Although the reason for this is unclear, it is considered as follows.

In the present invention, the UV scattering agent has moderate hydrophilicity because its hydrophobicity is equal to or less than a predetermined value. Further, the UV scattering agent contains dimethicone-treated zinc oxide microparticles, and the mass ratio of the content of the dimethicone-treated zinc oxide microparticles to the content of the UV scattering agent is equal to or greater than a predetermined value. The dimethicone-treated zinc oxide microparticles are well compatible with the aqueous-phase component, and can also be present in areas where the aqueous-phase component is present when applying the water-in-oil emulsified cosmetic, and the zinc oxide microparticles can be uniformly distributed on the skin surface; thus, it is considered that the UV protection effect over the entire skin can be improved, and that the wear resistance and water resistance of the cosmetic coating film can also be improved. Moreover, the aqueous-phase component has high wettability with the UV scattering agent because its surface tension is less than a predetermined value, and the content of the aqueous-phase component in the emulsified cosmetic of the present invention is within a predetermined range; thus, it is considered that the adhesion (liquid bridge adhesion) between the UV scattering agent and the skin surface caused by crosslinking of the aqueous-phase component can be improved. Furthermore, the amphiphilic substance which is solid at 25°C contained in the emulsified cosmetic of the present invention forms a stable structure in the oil phase, which is a continuous phase, and can contribute to the improvement of the stability of the entire formulation. It is considered that the synergistic effect of improving the liquid bridge adhesion and stabilizing the structure of the formulation can increase the yield value of the water-in-oil emulsified cosmetic. As a result, it is considered that the adhesion of the UV scattering agent to the skin surface is improved, that the UV scattering agent can be uniformly attached to skin ridges and skin grooves while preventing its flow from the skin ridges to the skin grooves, that the UV protection effect is improved, that when wearing a mask, even if some shear stress is applied to the cosmetic coating film formed on the skin surface due to friction between the mask and the cosmetic coating film, the UV scattering agent can be kept uniformly attached to the skin surface, and that wear resistance can be improved to suppress a decrease in the UV protection effect. Furthermore, as described above, the water-in-oil emulsified cosmetic has a high yield value, which improves the adhesion of the UV scattering agent to the skin surface, and it is considered to be possible to form a cosmetic coating film having excellent water resistance to be able to effectively prevent the UV protection components, such as the UV scattering agent and an optionally used UV absorber, from leaking into water, even if the cosmetic coating film on the skin surface gets wet with water.

<Component (A): UV scattering agent having a hydrophobicity degree of 73% or less in accordance with methanol titration>

The emulsified cosmetic of the present invention contains, as a component (A), an UV scattering agent having a hydrophobicity degree of 73% or less in accordance with methanol titration (hereinafter also referred to as "the component (A)"), from the viewpoint of scattering UV rays and improving the UV protection effect, and from the viewpoint of improving the wear resistance and water resistance of the cosmetic coating film.

The components (A) can be used singly or in combination of two or more.

The hydrophobicity degree of the component (A) in accordance with methanol titration is 73% or less, preferably 70% or less, more preferably 65% or less, and further more preferably 60% or less, and is preferably 30% or more, more preferably 35% or more, further more preferably 40% or more, and even more preferably 45% or more, from the viewpoint of increasing the yield value of the water-in-oil emulsified cosmetic, improving the adhesion of the component (A) to the skin surface, and improving the UV protection effect, and from the viewpoint of improving the wear resistance and water resistance of the cosmetic coating film. More specifically, the hydrophobicity degree of the component (A) in accordance with methanol titration is preferably from 30 to 70%, more preferably from 35 to 65%, further more preferably from 40 to 60%, and even more preferably from 45 to 60%. The hydrophobicity degree of the component (A) in accordance with methanol titration is determined as follows. Specifically, 50 mL of ion exchange water and 0.2 g of an UV scattering agent are placed in a beaker, and methanol is added dropwise from a burette while the mixture in the beaker is stirred with a magnetic stirrer. Due to the increase in the concentration of methanol in the beaker, the UV scattering agent settles, and the mixture in the beaker becomes cloudy to reduce transmittance. At this point, the mass fraction of methanol in the water/methanol mixed solution at which the transmittance of the mixture after dropping methanol is 50% of the transmittance of the mixture before dropping methanol is regarded as hydrophobicity (%). Specifically, the hydrophobicity degree of the component (A) is measured by the method described in the Examples.

The component (A) contains dimethicone-treated zinc oxide microparticles (A1) (hereinafter also referred to as "the component (A1)"), from the viewpoint of achieving uniform distribution on the skin surface and improving the UV protection effect, and from the viewpoint of improving the wear resistance and water resistance of the cosmetic coating film.

The component (A1) is zinc oxide microparticles hydrophobically treated with dimethicone as a surface treatment

agent. The dimethicone (cosmetic display name) as mentioned herein is a linear siloxane polymer in which dimethylpolysiloxane is end-capped with a trimethylsiloxy group.

The component (A1) may also contain a divalent or higher-valent metal. The component (A1) may contain metals, such as iron, zirconium, calcium, manganese, magnesium, and yttrium, or oxides thereof, singly or in combination of two or more.

The component (A) may further contain hydrophobically-treated metal oxide microparticles other than the component (A1), from the viewpoint of improving the UV protection effect, and from the viewpoint of improving the wear resistance and water resistance of the cosmetic coating film.

From the viewpoint of scattering UV rays and improving the UV protection effect, the hydrophobically-treated metal oxide microparticles other than the component (A1) are preferably one or more members selected from the group consisting of hydrophobically-treated zinc oxide microparticles, hydrophobically-treated titanium oxide microparticles, and hydrophobically-treated cerium oxide microparticles, all of which use a surface treatment agent other than dimethicone; and more preferably hydrophobically-treated titanium oxide microparticles (A2) (hereinafter also referred to as "the component (A2)"). The hydrophobically-treated metal oxide microparticles other than the component (A1) may contain a divalent or higher-valent metal. The hydrophobically-treated metal oxide microparticles may contain metals, such as iron, zirconium, calcium, manganese, magnesium, and yttrium, or oxides thereof, singly or in combination of two or more.

Examples of the hydrophobization treatment of the hydrophobically-treated metal oxide microparticles other than the component (A1) include silicone treatment; alkylalkoxysilane treatment; fatty acid treatment; fluorine-containing compound treatment using perfluoroalkyl phosphate, perfluoroalcohol, or the like; amino acid treatment using N-acylglutamic acid or the like; lecithin treatment; metal soap treatment; alkyl phosphate treatment; and ASI treatment using N-acylamino acid metal salt (sodium lauroyl aspartate), zinc chloride, and alkoxy titanium alkylate (isopropyl titanium triisostearate).

These surface treatments may be used singly or in combination of two or more.

Among these, from the viewpoint of adjusting hydrophobicity and improving the UV protection effect, and from the viewpoint of improving the wear resistance and water resistance of the cosmetic coating film, the hydrophobization treatment of the hydrophobically-treated metal oxide microparticles other than the component (A1) is preferably one or more members selected from the group consisting of silicone treatment, alkylalkoxysilane treatment, and fatty acid treatment.

Examples of the surface treatment agent used for silicone treatment include methylpolysiloxane, dimethylpolysiloxane (dimethicone), methylphenylpolysiloxane, methylhydrogenpolysiloxane (methicone), a dimethylsiloxane/-methylhydrogensiloxane copolymer (hydrogen dimethicone), methylcyclopolysiloxane, dodecamethylcyclohexasiloxane, tetradecamethylhexasiloxane, a dimethylsiloxane/methyl(polyoxyethylene)siloxane/methyl(p olyoxypropylene)siloxane copolymer, a dimethylsiloxane/methyl(polyoxyethylene)siloxane copolymer, a dimethylsiloxane/methyl(polyoxypropylene)siloxane copolymer, a dimethylsiloxane/methylcetyloxysiloxane copolymer, a dimethylsiloxane/methylstearoxysiloxane copolymer, an (alkyl acrylate/dimethicone) copolymer, and other various silicone oils. Among these, from the viewpoint of adjusting hydrophobicity and improving the UV protection effect, and from the viewpoint of improving the wear resistance and water resistance of the cosmetic coating film, the surface treatment agent used for silicone treatment is more preferably one or more members selected from the group consisting of methylpolysiloxane, dimethylpolysiloxane (dimethicone), methylhydrogenpolysiloxane, and a dimethylsiloxane/methylhydrogensiloxane copolymer (hydrogen dimethicone).

From the viewpoint of adjusting hydrophobicity and improving the UV protection effect, and from the viewpoint of improving the wear resistance and water resistance of the cosmetic coating film, the surface treatment agent used for alkylalkoxysilane treatment is preferably an alkylalkoxysilane having a linear or branched alkyl group having 6 or more and 20 or less carbon atoms, and more preferably one or more members selected from the group consisting of octyltriethoxysilane (triethoxycaprylylsilane) and octyltrimethoxysilane (trimethoxycaprylylsilane).

Preferred examples of the surface treatment agent used for fatty acid treatment include linear or branched higher fatty acids having 12 or more and 22 or less carbon atoms. Among these, from the viewpoint of adjusting hydrophobicity and improving the UV protection effect, and from the viewpoint of improving the wear resistance and water resistance of the cosmetic coating film, the surface treatment agent used for fatty acid treatment is more preferably a linear or branched higher fatty acid having 14 or more and 22 or less carbon atoms, further more preferably a linear or branched higher fatty acid having 16 or more and 20 or less carbon atoms, and even more preferably one or more members selected from the group consisting of stearic acid and isostearic acid.

As described above, from the viewpoint of adjusting hydrophobicity and improving the UV protection effect, and from the viewpoint of improving the wear resistance and water resistance of the cosmetic coating film, the hydrophobization treatment of the hydrophobically-treated metal oxide microparticles other than the component (A1) is more preferably one or more members selected from the group consisting of silicone treatment using, as a surface treatment agent, one or more members selected from the group consisting of methylpolysiloxane, dimethylpolysiloxane (dimethicone), methylhydrogenpolysiloxane, and a dimethylsiloxane/methylhydrogensiloxane copolymer (hydrogen dimethicone), alkylalkoxysilane treatment using, as a surface treatment agent, an alkylalkoxysilane having a linear or branched alkyl group having 6 or more and 20 or less carbon atoms, and fatty acid treatment using, as a surface treatment agent, a linear or branched higher fatty acid having 14 or more and 22 or less carbon atoms; and further more preferably fatty acid treatment using, as a surface treatment agent, a linear or branched higher fatty acid having 14 or more and 22 or less carbon atoms.

The surface treatment agents mentioned above may be used singly or in combination of two or more.

When the hydrophobically-treated titanium oxide microparticles (A2) are used as the hydrophobically-treated metal oxide microparticles other than the component (A1), from the viewpoint of reducing photocatalytic activity, surface treatment agents, such as hydrous oxides of metals, such as silica, hydrous silica, and aluminum, may also be used in combination, in addition to the surface treatment agent used for the hydrophobization treatment mentioned above.

The amount of surface treatment in the component (A) by hydrophobization treatment (hereinafter also referred to as "the hydrophobic surface treatment amount") is preferably 0.1 mass% or more, more preferably 0.5 mass% or more, and further more preferably 1 mass% or more, and is preferably 40 mass% or less, more preferably 30 mass% or less, further more preferably 20 mass% or less, and even more preferably 10 mass% or less, from the viewpoint of adjusting hydrophobicity and improving the UV protection effect, and from the viewpoint of improving the wear resistance and water resistance of the cosmetic coating film.

In the present invention, when the component (A) is hydrophobically-treated metal oxide microparticles, the hydrophobicity, mass, and average primary particle size, described later, of the component (A) refer to the hydrophobicity, mass, and average primary particle size thereof including the surface treatment agent.

The shape of the component (A) may be, for example, spherical, flaky, plate-like, rod-like, spindle-like, needle-like, or irregular, but any shape can be used.

The average primary particle size of the component (A) is preferably 3 nm or more, more preferably 5 nm or more, and further more preferably 10 nm or more, and is preferably 800 nm or less, more preferably 700 nm or less, further more preferably 500 nm or less, even more preferably 300 nm or less, even more preferably 100 nm or less, even more preferably 50 nm or less, and even more preferably 40 nm or less, from the viewpoint of adjusting hydrophobicity and improving the UV protection effect, from the viewpoint of improving the wear resistance and water resistance of the cosmetic coating film, and from the viewpoint of versatility. More specifically, the average primary particle size of the component (A) is preferably from 3 nm to 800 nm, more preferably from 5 to 700 nm, further more preferably from 5 to 500 nm, even more preferably from 5 to 300 nm, even more preferably from 5 to 100 nm, even more preferably from 5 to 50 nm, even more preferably from 10 to 50 nm, and even more preferably from 10 to 40 nm, from the same viewpoint as above.

The average primary particle size of the component (A) in the present invention can be determined from images observed with a transmission electron microscope (TEM). Specifically, the average primary particle size can be determined by observing the particle size at a magnification of 50 000 times using a TEM, measuring the maximum minor axes of 300 primary particles in the observed image, and calculating the number average value thereof. When the component (A) has a shape other than a flaky or plate-like shape, the maximum minor axis as mentioned herein refers to, among the minor axes perpendicular to the major axes, a minor axis having the maximum length. When the component (A) has a flaky or plate-like shape, the average primary particle size is determined by measuring the thicknesses of 300 primary particles in an image observed under the same conditions as above, and calculating the number average value thereof. Specifically, the average primary particle size is measured by the method described in the Examples.

The component (A) can be prepared by treating metal oxide microparticles before hydrophobization treatment as a component (Ax) by a known method using any of the surface treatment agents mentioned above. For example, as surface treatment using the silicone oil mentioned above, as described in JP-B-3187440, the component (A) can be prepared by coating metal oxide microparticles, such as zinc oxide microparticles, with at least one silicone compound (excluding silane compounds) selected from the group consisting of organopolysiloxanes and silicone resins in a non-gaseous phase, and firing the resulting microparticles at a temperature of from 600 to 950°C in an oxygen-containing atmosphere, thereby coating the surface of the metal oxide microparticles with silicon oxide. As surface treatment using the alkylalkoxysilane mentioned above, as described in JP-A-2007-326902, the component (A) can be prepared by coating metal oxide microparticles with a specific polysiloxane compound, followed by surface treatment with the alkylalkoxysilane in water.

The preferred embodiment and measurement method of the average primary particle size of the component (Ax) (metal oxide microparticles before hydrophobization treatment) used in the hydrophobization treatment mentioned above are the same as the preferred embodiment and measurement method for the component (A).

Commercial products of the dimethicone-treated zinc oxide microparticles used as the component (A1) include MZ series (manufactured by Tayca Corporation).

Commercial products of the hydrophobically-treated titanium oxide microparticles used as the component (A2) include MT series and MTY series (both manufactured by Tayca Corporation); STR series (manufactured by Sakai Chemical Industry Co., Ltd.); and TTO-55 series and TTO-51 series (both manufactured by Ishihara Sangyo Kaisha, Ltd.).

The content of the component (A) in the emulsified cosmetic of the present invention is preferably 5 mass% or more, more preferably 10 mass% or more, further more preferably 15 mass% or more, even more preferably 20 mass% or

more, and even more preferably 25 mass% or more, from the viewpoint of improving the UV protection effect, and is preferably 40 mass% or less, more preferably 37 mass% or less, further more preferably 35 mass% or less, and even more preferably 30 mass% or less, from the viewpoint of improving emulsion stability, from the viewpoint of the effect of improving the stratum corneum through continuous application, and from the viewpoint of reducing the whiteness of the cosmetic coating film for a natural look. More specifically, the content of the component (A) in the emulsified cosmetic of the present invention is preferably from 5 to 40 mass%, more preferably from 10 to 37 mass%, further more preferably from 15 to 35 mass%, even more preferably from 20 to 35 mass%, even more preferably from 25 to 35 mass%, and even more preferably from 25 to 30 mass%, from the viewpoint of enhancing the UV protection effect, from the viewpoint of improving emulsion stability, and from the viewpoint of the effect of improving the stratum corneum through continuous application.

The mass ratio of the content of the component (A1) to the content of the component (A) in the emulsified cosmetic of the present invention [component (A1)/component (A)] is 0.38 or more, preferably 0.50 or more, more preferably 0.60 or more, further more preferably 0.63 or more, and even more preferably 0.65 or more, from the viewpoint of improving the UV protection effect, and is preferably 1.0 or less, more preferably 0.80 or less, further more preferably 0.76 or less, even more preferably 0.72 or less, and even more preferably 0.70 or less, from the viewpoint of the effect of improving the stratum corneum through continuous application, and from the viewpoint of reducing the whiteness of the cosmetic coating film for a natural look. More specifically, the mass ratio [component (A1)/component (A2)] is preferably from 0.38 to 1.0, more preferably from 0.38 to 0.80, further more preferably from 0.38 to 0.76, even more preferably from 0.50 to 0.76, even more preferably from 0.60 to 0.76, even more preferably from 0.63 to 0.76, even more preferably from 0.63 to 0.72, and even more preferably from 0.65 to 0.70, from the viewpoint of enhancing the UV protection effect, from the viewpoint of the effect of improving the stratum corneum through continuous application, and from the viewpoint of reducing the whiteness of the cosmetic coating film for a natural look.

When the component (A) further contains hydrophobically-treated titanium oxide microparticles (A2), the mass ratio of the content of the dimethicone-treated zinc oxide microparticles (A1) to the content of the hydrophobically-treated titanium oxide microparticles (A2) in the emulsified cosmetic of the present invention [dimethicone-treated zinc oxide microparticles (A1)/hydrophobically-treated titanium oxide microparticles (A2)] (hereinafter also referred to as "the mass ratio [component (A1)/component (A2)]") is preferably 0.50 or more, more preferably 1.0 or more, further more preferably 1.5 or more, and even more preferably 2.0 or more, and is preferably 3.5 or less, more preferably 3.4 or less, further more preferably 3.3 or less, and even more preferably 3.2 or less. More specifically, the mass ratio [component (A1)/component (A2)] is preferably from 0.50 to 3.5, more preferably from 1.0 to 3.4, further more preferably from 1.5 to 3.3, and even more preferably from 2.0 to 3.2.

When the component (A) further contains hydrophobically-treated titanium oxide microparticles (A2), the content of the component (A2) in the water-in-oil emulsified cosmetic of the present invention is preferably less than 10 mass%, more preferably 9 mass% or less, and further more preferably 8 mass% or less, from the viewpoint of reducing the whiteness of the cosmetic coating film for a natural look, and is preferably 1 mass% or more, more preferably 3 mass% or more, and further more preferably 5 mass% or more, from the viewpoint of improving the UV protection effect, and from the viewpoint of improving the wear resistance and water resistance of the cosmetic coating film. More specifically, the content of the component (A2) in the water-in-oil emulsified cosmetic of the present invention is preferably from 1 to 9 mass%, more preferably from 3 to 9 mass%, further more preferably from 5 to 9 mass%, and even more preferably from 5 to 8 mass%, from the viewpoint of improving the UV protection effect, from the viewpoint of improving the wear resistance and water resistance of the cosmetic coating film, and from the viewpoint of reducing the whiteness of the cosmetic coating film for a natural look.

<Component (B): Amphiphilic substance which is solid at 25°C>

The emulsified cosmetic of the present invention contains, as a component (B), an amphiphilic substance which is solid at 25°C (hereinafter also referred to as "the component (B)"), from the viewpoint of improving the UV protection effect, and from the viewpoint of improving the wear resistance and water resistance of the cosmetic coating film. In the present invention, the definition of "solid" is as described above.

For example, the component (B) is preferably one or more members selected from the group consisting of ceramide, an alcohol having 8 or more and 22 or less carbon atoms, a dextrin fatty acid ester having 8 or more and 22 or less carbon atoms, a glycerin fatty acid ester having 8 or more and 22 or less carbon atoms, an alkyl glyceryl ether having an alkyl group having 8 or more and 22 or less carbon atoms, a sorbitan fatty acid ester having 8 or more and 22 or less carbon atoms, and a polyoxyethylene sorbitan fatty acid ester having 8 or more and 22 or less carbon atoms.

The components (B) can be used singly or in combination of two or more.

[Ceramide]

The ceramide can be one or more members selected from the group consisting of natural ceramides and pseudo-ceramides. From the viewpoint of stably dispersing the component (A) and improving the UV protection effect, and from the viewpoint of improving the wear resistance and water resistance of the cosmetic coating film, the ceramides described in JP-A-2013-53146 are preferred.

Specific examples of natural ceramides include Ceramide Types 1 to 7 in which sphingosine, dihydrosphingosine, phytosphingosine, or sphingadienine is amidated (e.g., the porcine and human ceramides shown in Fig. 2 of J. Lipid Res., 24: 759 (1983) and Fig. 4 of J. Lipid. Res., 35: 2069 (1994)). Further, N-alkylated forms (e.g., N-methylated forms) of these ceramides are also included.

These ceramides may be in the form of natural (D(-)) optically active forms, non-natural (L(+)) optically active forms, or mixtures thereof. The relative configuration of these compounds is not particularly limited, and may be a natural configuration, a non-natural configuration, or a mixture thereof. Particularly preferred are the compounds of CERAMIDE 1, CERAMIDE 2, CERAMIDE 3, CERAMIDE 5, and CERAMIDE 6II (all INCI, 8th Edition), and those of the following formulas.

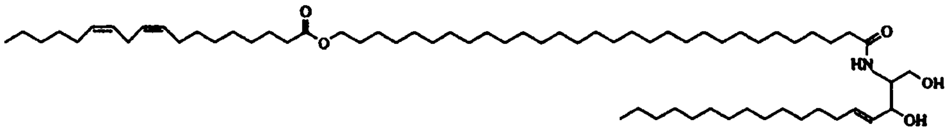

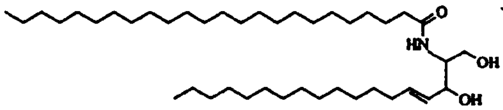

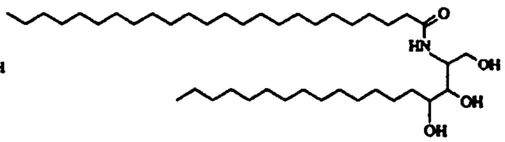

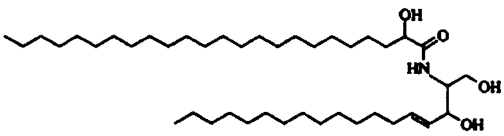

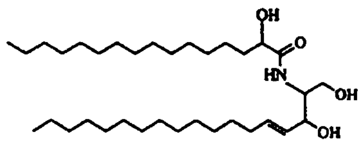

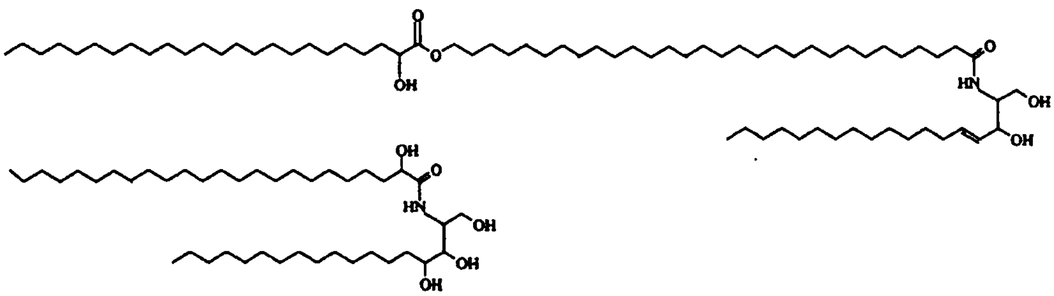

Commercially available natural ceramides include Ceramide I, Ceramide III, Ceramide IIIA, Ceramide IIIB, Ceramide IIIC, and Ceramide VI (all manufactured by Cosmo Farm), Ceramide TIC-001 (Takasago International Corporation), CERAMIDE II (Quest International), DS-Ceramide VI, DS-CLA-Phytoceramide, C6-Phytoceramide, and DS-ceramide Y3S (Doosan), and CERAMIDE 2 (Sederma).

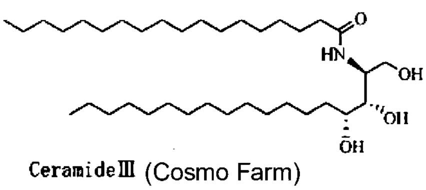

CeramideIII (Cosmo Farm)

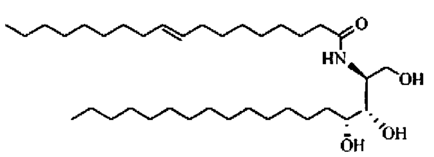

CeramideIIIB (Cosmo Farm)

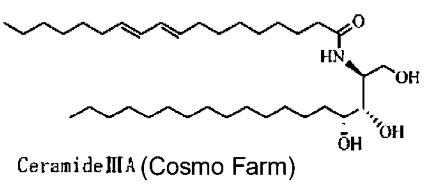

CeramideIIIA (Cosmo Farm)

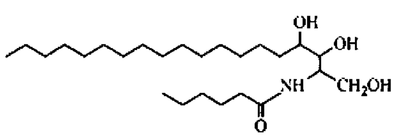

Phytoceramide (Doosan)

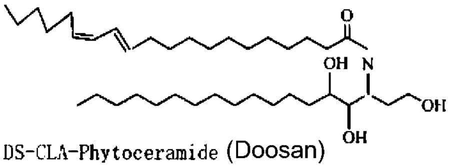

DS-CLA-Phytoceramide (Doosan)

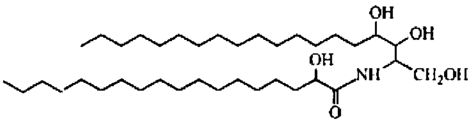

DS-Ceramide VI (Doosan)

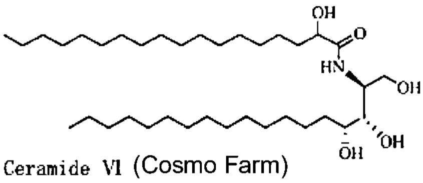

Ceramide VI (Cosmo Farm)

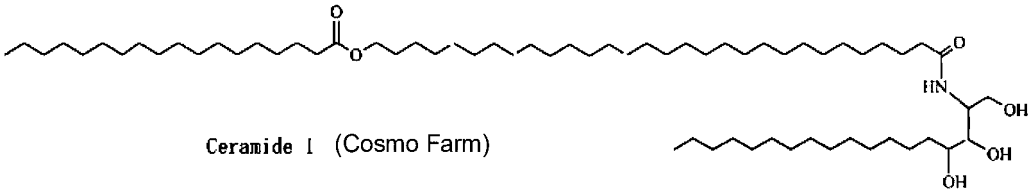

Ceramide I (Cosmo Farm)

The pseudo-ceramide is preferably pseudo-ceramide of the following formula (1), from the viewpoint of stably dispersing the component (A) and improving the UV protection effect, and from the viewpoint of improving the wear resistance and water resistance of the cosmetic coating film.

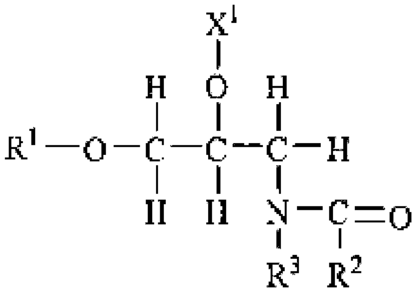

(1)

In the formula (1), $R^1$ represents a linear, branched, or cyclic hydrocarbon group having 10 or more and 22 or less carbon atoms and optionally substituted with a hydroxyl group, or a hydrogen atom; $X^1$ represents a hydrogen atom, an acetyl group, or a glyceryl group; $R^2$ represents a linear, branched, or cyclic hydrocarbon group having 5 or more and 22 or less carbon atoms and optionally substituted with a hydroxyl group or amino group, or a group in which a linear or branched fatty acid having 8 or more and 22 or less carbon atoms and optionally substituted with a hydroxy group is ester-bonded to the ω-end of the hydrocarbon group; and $R^3$ represents an alkyl group having a total of 1 or more and 30 or less carbon atoms and optionally substituted with a hydrogen atom, a hydroxy group, a hydroxyalkoxy group, an alkoxy group, or an acetoxy groups.

Among these, from the viewpoint of stably dispersing the component (A) and improving the UV protection effect, and from the viewpoint of improving the wear resistance and water resistance of the cosmetic coating film, the pseudo-ceramides of the following formulas are more preferred.

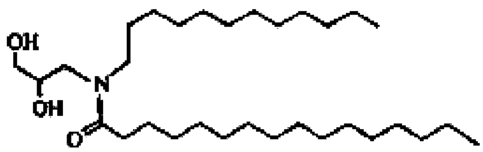

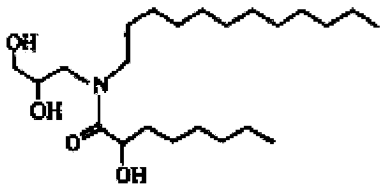

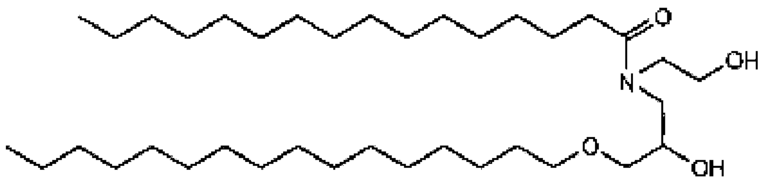

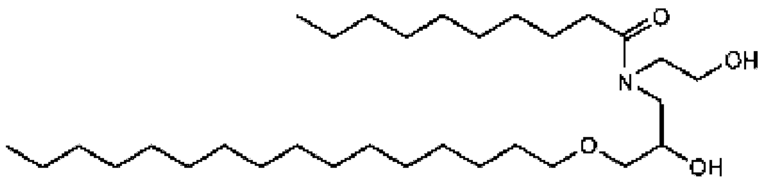

[Alcohol having 8 or more and 22 or less carbon atoms]

Examples of the alcohol having 8 or more and 22 or less carbon atoms include those having a linear or branched alkyl group or alkenyl group having 8 or more and 22 or less carbon atoms. Examples of such alcohols include lauryl alcohol, myristyl alcohol, cetyl alcohol, stearyl alcohol, behenyl alcohol, oleyl alcohol, and the like. Among these, from the viewpoint of stably dispersing the component (A) and improving the UV protection effect, and from the viewpoint of improving the wear resistance and water resistance of the cosmetic coating film, preferred is an alcohol having a linear alkyl group, more preferred is an alcohol having 16 or more and 18 or less carbon atoms, and further more preferred is one or more members selected from the group consisting of cetyl alcohol and stearyl alcohol.

[Dextrin fatty acid ester having 8 or more and 22 or less carbon atoms]

The dextrin fatty acid ester having 8 or more and 22 or less carbon atoms is an ester of fatty acid having 8 or more and 22 or less carbon atoms and dextrin. Examples of the dextrin fatty acid ester include dextrin palmitate, dextrin stearate, dextrin palmitate/stearate, dextrin oleate, dextrin isopalmitate, dextrin isostearate, dextrin myristate, dextrin palmitate/2-ethylhexanoate, and the like. Among these, from the viewpoint of stably dispersing the component (A) and improving the UV protection effect, and from the viewpoint of improving the wear resistance and water resistance of the cosmetic coating film, preferred is one or more members selected from the group consisting of dextrin palmitate, dextrin myristate, and dextrin palmitate/2-ethylhexanoate, and more preferred is dextrin palmitate.

[Glycerin fatty acid ester having 8 or more and 22 or less carbon atoms]

The glycerin fatty acid ester having 8 or more and 22 or less carbon atoms is an ester of fatty acid having 8 or more and 22 or less carbon atoms and glycerin. Examples of the glycerin fatty acid ester include glycerin monocaprylate, glycerin monolaurate, glycerin monomyristate, glycerin monopalmitate, glycerin monostearate, glycerin monobehenate, glycerin monooleate, glycerin monoisostearate, glycerin dicaprylate, and the like. Among these, preferred is one or more members selected from the group consisting of glycerin monocaprylate, glycerin monooleate, glycerin monobehenate, glycerin monostearate, and glycerin monopalmitate; and more preferred is one or more members selected from the group consisting of glycerin monocaprylate, glycerin monooleate, and glycerin monostearate.

[Alkyl glyceryl ether having an alkyl group having 8 or more and 22 or less carbon atoms]

Examples of the alkyl glyceryl ether having an alkyl group having 8 or more and 22 or less carbon atoms include monodecyl glyceryl ether, monolauryl glyceryl ether, monomyristyl glyceryl ether, monocetyl glyceryl ether, monostearyl glyceryl ether, monobehenyl glyceryl ether, and the like.

[Sorbitan fatty acid ester having 8 or more and 22 or less carbon atoms]

The sorbitan fatty acid ester having 8 or more and 22 or less carbon atoms is preferably a monoester or diester of fatty acid having 8 or more and 22 or less carbon atoms and sorbitan. Examples of the sorbitan fatty acid ester include sorbitan monolaurate, sorbitan monomyristate, sorbitan monopalmitate, sorbitan monostearate, sorbitan monooleate, sorbitan monobehenate, sorbitan dioleate, and the like.

[Polyoxyethylene sorbitan fatty acid ester having 8 or more and 22 or less carbon atoms]

Examples of the polyoxyethylene sorbitan fatty acid ester having 8 or more and 22 or less carbon atoms include polyoxyethylene sorbitan monolaurate, polyoxyethylene sorbitan monomyristate, polyoxyethylene sorbitan monopalmitate, polyoxyethylene sorbitan monostearate, polyoxyethylene sorbitan monobehenate, and the like.

Among these, from the viewpoint of stably dispersing the component (A) and improving the UV protection effect, from the viewpoint of improving the wear resistance and water resistance of the cosmetic coating film, and from the viewpoint of enhancing the effect of improving the stratum corneum through continuous application, the component (B) is more preferably one or more members selected from the group consisting of ceramide and a dextrin fatty acid ester having 8 or more and 22 or less carbon atoms, further more preferably a dextrin fatty acid ester having 8 or more and 22 or less carbon atoms, and even more preferably a combination of ceramide (hereinafter also referred to as "the component (b1)") and a dextrin fatty acid ester having 8 or more and 22 or less carbon atoms (hereinafter also referred to as "the component (b2)"). Due to the combined use of ceramide (component (b1)) and a dextrin fatty acid ester having 8 or more and 22 or less carbon atoms (component (b2)) as the component (B), the component (b1) and the component (b2) form a co-crystalline structure, which is considered to be able to further improve the stability of the entire formulation, to increase the yield value of the water-in-oil emulsified cosmetic, to improve the adhesion of the component (A) to the skin surface, and to improve the UV protection effect, the wear resistance and water resistance of the cosmetic coating film, and the effect of improving the stratum corneum through continuous application.

The content of the component (B) in the emulsified cosmetic of the present invention is preferably 0.1 mass% or more, more preferably 0.5 mass% or more, further more preferably 1 mass% or more, even more preferably 2 mass% or more, and even more preferably 3 mass% or more, from the viewpoint of stably dispersing the component (A) and improving the UV protection effect, from the viewpoint of improving the wear resistance and water resistance of the cosmetic coating film, and from the viewpoint of enhancing the effect of improving the stratum corneum through continuous application, and is preferably 12 mass% or less, more preferably 10 mass% or less, and further more preferably 9 mass% or less, from the viewpoint of improving emulsion stability. More specifically, the content of the component (B) in the emulsified cosmetic of the present invention is preferably from 0.1 to 12 mass%, more preferably from 0.5 to 12 mass%, further more preferably from 1 to 10 mass%, even more preferably from 2 to 10 mass%, even more preferably from 2 to 9 mass%, and even more preferably from 3 to 9 mass%, from the same viewpoints as above.

When ceramide (component (b1)) and a dextrin fatty acid ester having 8 or more and 22 or less carbon atoms (component (b2)) are used in combination as the component (B), the mass ratio of the content of the ceramide (component (b1)) to the content of the dextrin fatty acid ester (component (b2)) in the emulsified cosmetic of the present invention [component (b1)/component (b2)] is preferably 0.5 or more, more preferably 1 or more, further more preferably 3 or more, even more preferably 5 or more, and even more preferably 7 or more, and is preferably 30 or less, more preferably 20 or less, further more preferably 15 or less, and even more preferably 13 or less, from the viewpoint of increasing the yield value of the water-in-oil emulsified cosmetic, improving the adhesion of the component (A) to the skin surface, and improving the UV protection effect, from the viewpoint of improving the wear resistance and water resistance of the cosmetic coating film, and from the viewpoint of enhancing the effect of improving the stratum corneum through continuous application. More specifically, the mass ratio [component (b1)/component (b2)] is preferably from 0.5 to 30, more preferably from 1 to 20, further more preferably from 3 to 15, even more preferably from 5 to 13, and even more preferably from 7 to 13, from the same viewpoints as above.

The mass ratio of the content of the component (A) to the content of the component (B) in the emulsified cosmetic of the present invention [component (A)/component (B)] is preferably 1 or more, more preferably 2 or more, and further more preferably 3 or more, from the viewpoint of stably dispersing the component (A) and improving the UV protection effect, and from the viewpoint of improving the wear resistance and water resistance of the cosmetic coating film, and is preferably 100 or less, more preferably 70 or less, further more preferably 50 or less, even more preferably 30 or less, even more preferably 25 or less, even more preferably 20 or less, even more preferably 15 or less, and even more preferably 12 or less, from the viewpoint of enhancing the effect of improving the stratum corneum through continuous application. More specifically, the mass ratio [component (A)/component (B)] is preferably from 1 to 100, more preferably from 1 to 70, further more preferably from 1 to 50, even more preferably from 1 to 30, even more preferably from 2 to 25, even more preferably from 3 to 20, even more preferably from 3 to 15, and even more preferably from 3 to 12, from the same viewpoints as above.

<Component (B'): Amphiphilic substance which is liquid at 25°C>

The emulsified cosmetic of the present invention preferably further contains an amphiphilic substance which is liquid at 25°C (hereinafter also referred to as "the component (B')"), from the viewpoint of preventing an increase in the viscosity of the water-in-oil emulsified cosmetic and improving the use impression. In the present invention, the definition of "liquid" is as described above.

When the emulsified cosmetic of the present invention further contains the component (B'), the component (C) is a component other than the component (B) and the component (B').

For example, the component (B') is preferably an oil-soluble surfactant having an HLB of 14.5 or less. The HLB (hydrophile-lipophile balance) of the component (B') as mentioned herein is an index which expresses the ratio of the relative affinity of a surfactant for both liquids in an oil-water system, and can be determined by the Griffin method (J. Soc. Cosm. Chem., 1954, 5: 249-256) from the following formula:

HLB = 20 × [(molecular weight of hydrophilic group contained in surfactant)/(molecular weight of surfactant)]

Examples of the hydrophilic group contained in the surfactant include a hydroxy group and an ethyleneoxy group.

The component (B') is more preferably a nonionic surfactant, from the viewpoint of improving emulsion stability and improving the use impression.

A specific example of the component (B') is further more preferably one or more members selected from the group consisting of polyether-modified silicone, polyglycerin-modified silicone, acrylic silicone, a sorbitan fatty acid ester, a glycerin fatty acid ester, and a polyoxyethylene hydrogenated castor oil. Among these, from the same viewpoints as above, the component (B') is even more preferably one or more members selected from the group consisting of polyether-modified silicone, polyglycerin-modified silicone, and acrylic silicone; and even more preferably polyether-modified silicone.

The fatty acids in the sorbitan fatty acid ester and glycerin fatty acid ester preferably have 8 or more and 22 or less carbon atoms, more preferably 10 or more and 20 or less carbon atoms, and further more preferably 12 or more and 18 or less carbon atoms.

The polyether-modified silicone has a structure in which the side chain and/or terminal hydrocarbon groups of silicone oil are replaced with polyether groups.

The polyether group of the polyether-modified silicone is preferably a polyethyleneoxy group, a polypropyleneoxy group, or a polyalkyleneoxy group in which an ethyleneoxy group (EO) and a propyleneoxy group (a trimethyleneoxy group or a propane-1,2-diyloxy group; PO) are added in blocks or randomly, and more preferably a polyethyleneoxy group.

The polyether-modified silicone can be a compound with a polyether group grafted onto a silicone main chain, a compound in which silicone and a polyether group are bonded in blocks, or the like.

The content of the component (B') in the emulsified cosmetic of the present invention is preferably 0.5 mass% or more, more preferably 1 mass% or more, and further more preferably 2 mass% or more, from the viewpoint of preventing an increase in the viscosity of the water-in-oil emulsified cosmetic and improving the use impression, and is preferably 5 mass% or less, more preferably 4 mass% or less, and further more preferably 3 mass% or less, from the viewpoint of improving the stability of the entire formulation without impairing the formation of a stable structure in the oil phase by the component (B), and improving the UV protection effect. More specifically, the content of the component (B') in the emulsified cosmetic of the present invention is preferably from 0.5 to 5 mass%, more preferably from 1 to 4 mass%, and further more preferably from 2 to 3 mass%, from the same viewpoints as above.

When the emulsified cosmetic of the present invention further contains an amphiphilic substance which is liquid at 25°C as the component (B'), the mass ratio of the content of the component (B') to the content of the component (B) in the emulsified cosmetic of the present invention [component (B')/component (B)] is preferably 0.10 or more, more preferably 0.20 or more, and further more preferably 0.30 or more, from the viewpoint of preventing an increase in the viscosity of the water-in-oil emulsified cosmetic and improving the use impression, and is preferably 12 or less, more preferably 8 or less, further more preferably 6 or less, even more preferably 4 or less, even more preferably 2 or less, and even more preferably 1 or less, from the viewpoint of improving the stability of the entire formulation without impairing the formation of a stable structure in the oil phase by the component (B), and improving the UV protection effect. More specifically, the mass ratio [component (B')/component (B)] is preferably from 0.10 to 12, more preferably from 0.20 to 8, further more preferably from 0.30 to 6, even more preferably from 0.30 to 4, even more preferably from 0.30 to 2, and even more preferably from 0.30 to 1, from the same viewpoints as above.

When the emulsified cosmetic of the present invention further contains an amphiphilic substance which is liquid at 25°C as the component (B') and a dextrin fatty acid ester having 8 or more and 22 or less carbon atoms (b2) as the component (B), the mass ratio of the content of the component (B') to the content of the component (b2) in the emulsified

cosmetic of the present invention [component (B')/component (b2)] is preferably 3 or more, more preferably 4 or more, further more preferably 6 or more, and even more preferably 8 or more, from the viewpoint of preventing an increase in the viscosity of the water-in-oil emulsified cosmetic and improving the use impression, and is preferably 12 or less, more preferably 11 or less, and further more preferably 10 or less, from the viewpoint of improving the stability of the entire formulation without impairing the formation of a stable structure in the oil phase by the component (B), and improving the UV protection effect. More specifically, the mass ratio [component (B')/component (b2)] is preferably from 3 to 12, more preferably from 3 to 11, further more preferably from 4 to 11, even more preferably from 6 to 10, and even more preferably from 8 to 10, from the same viewpoints as above.

<Component (C): Aqueous-phase component>

The emulsified cosmetic of the present invention contains, as a component (C), an aqueous-phase component (hereinafter also referred to as "the component (C)") (provided that the component (B) is excluded), from the viewpoint of improving the adhesion of the component (A) to the skin surface and improving the UV protection effect, and from the viewpoint of improving the wear resistance and water resistance of the cosmetic coating film.

In the present invention, the "aqueous-phase component" refers to water and a water-soluble component (provided that the component (B) is excluded) as described above, and the "water-soluble component" refers to a component which has a uniform appearance when visually observed in a mixture obtained by mixing 100 parts by mass of water and 3 parts by mass of the component at 25°C. The phrase that "the mixture has a uniform appearance" as mentioned herein means that no separation is visually observed, and that the mixture is transparent or opaque.

The water-soluble components can be used singly or in combination of two or more.

When the emulsified cosmetic of the present invention contains a component (D) described later, the component (D) is not included in the component (C). That is, when the emulsified cosmetic of the present invention contains a component (D) described later, the component (C) is water and a water-soluble component (provided that the component (B) and the component (D) are excluded).

The component (C) preferably contains one or more members selected from the group consisting of water, an alcohol (hereinafter also referred to as "the alcohol (C1)"), and a surfactant (provided that the component (B) and the component (B') are excluded) (hereinafter also referred to as "the surfactant (C2)"), and more preferably contains water, an alcohol (C1), and a surfactant (C2), from the viewpoint of obtaining good emulsion stability.

When the emulsified cosmetic of the present invention further contains a surfactant (C2), the surfactant (C2) is treated as the component (C).

Usable examples of water include deionized water, distilled water, highly pure water, and ultrapure purified water.

[Alcohol (C1)]

The alcohol (C1) is preferably one or more members selected from the group consisting of monohydric alcohols and polyhydric alcohols, from the viewpoint of improving the adhesion of the component (A) to the skin surface and improving the UV protection effect, and from the viewpoint of improving the wear resistance and water resistance of the cosmetic coating film.

Examples of monohydric alcohols include saturated monohydric alcohols having 2 or more and 4 or less carbon atoms, such as ethanol, propanol, isopropanol, and t-butyl alcohol.

Examples of polyhydric alcohols include divalent alcohols, such as ethylene glycol, diethylene glycol, triethylene glycol, polyethylene glycol (an average molecular weight of less than 650), 1,2-propanediol (propylene glycol), 1,3-propanediol (propanediol), dipropylene glycol, polypropylene glycol (an average molecular weight of less than 650), 1,3-butanediol (1,3-butylene glycol), and polybutylene glycol (an average molecular weight of less than 650); and trivalent alcohols, such as glycerin, diglycerin, polyglycerin.

Among these, from the viewpoint of improving the adhesion of the component (A) to the skin surface and improving the UV protection effect, and from the viewpoint of improving the wear resistance and water resistance of the cosmetic coating film, the alcohol (C1) is more preferably one or more members selected from the group consisting of saturated monohydric alcohols having 2 or more and 4 or less carbon atoms and polyhydric alcohols having 2 or more and 12 or less carbon atoms; further more preferably one or more members selected from the group consisting of ethanol, ethylene glycol, diethylene glycol, 1,2-propanediol (propylene glycol), 1,3-propanediol (propanediol), dipropylene glycol, 1,3-butanediol (1,3-butylene glycol), and glycerin; even more preferably one or more members selected from the group consisting of ethanol, 1,3-propanediol, dipropylene glycol, 1,3-butanediol, and glycerin; even more preferably one or more members selected from the group consisting of ethanol, dipropylene glycol, 1,3-butanediol, and glycerin; and even more preferably one or more members selected from the group consisting of dipropylene glycol, 1,3-butanediol, and glycerin.

The content of the alcohol (C1) in the emulsified cosmetic of the present invention is preferably 3 mass% or more, more preferably 5 mass% or more, and further more preferably 7 mass% or more, and is preferably 40 mass% or less, more

preferably 35 mass% or less, and further more preferably 30 mass% or less, from the viewpoint of improving the adhesion of the component (A) to the skin surface and improving the UV protection effect, and from the viewpoint of improving the wear resistance and water resistance of the cosmetic coating film. More specifically, the content of the alcohol (C1) in the emulsified cosmetic of the present invention is preferably from 3 to 40 mass%, more preferably from 5 to 35 mass%, and further more preferably from 7 to 30 mass%, from the same viewpoints as above.

[Surfactant (C2)]

The surfactant (C2) is not particularly limited as long as it is a water-soluble surfactant other than the component (B) and the component (B'), and any of nonionic, anionic, cationic, and amphoteric surfactants can be used. From the viewpoint of obtaining good emulsion stability, nonionic surfactants are preferred. The surfactants (C2) can be used singly or in combination of two or more. Among these, the surfactant (C2) is more preferably one or more members selected from the group consisting of a polyoxyalkylene fatty acid ester, a polyoxyalkylene alkyl ether, a polyoxyalkylene alkenyl ether, an alkyl glucoside, a sucrose fatty acid ester, a polyglycerin fatty acid ester, a polyoxyalkylene hydrogenated castor oil, an alkyl saccharide, an alkylamine oxide, and an alkyl amidoamine oxide, from the viewpoint of obtaining good emulsion stability.

The alkyl groups in the polyoxyalkylene alkyl ether, alkyl glucoside, alkyl saccharide, alkylamine oxide, and alkyl amidoamine oxide, the alkenyl group in the polyoxyalkylene alkenyl ether, and the fatty acids in the polyoxyalkylene fatty acid ester, sucrose fatty acid ester, and polyglycerin fatty acid ester preferably have 8 or more and 22 or less carbon atoms, more preferably 10 or more and 20 or less carbon atoms, and further more preferably 12 or more and 18 or less carbon atoms, from the viewpoint of obtaining good emulsion stability.

Among these, the surfactant (C2) is further more preferably a polyoxyalkylene alkyl ether, and even more preferably a polyoxyalkylene alkyl ether having an alkyl group having 8 or more and 22 or less carbon atoms, from the viewpoint of obtaining good emulsion stability.

From the viewpoint of obtaining good emulsion stability, the HLB of the surfactant (C2) is preferably 8 or more, more preferably 10 or more, and further more preferably 12 or more, and is preferably 18 or less, more preferably 17 or less, and further more preferably 16 or less. More specifically, the HLB of the component (C2) is preferably from 8 to 18, more preferably from 10 to 17, and further more preferably from 12 to 16.

The HLB (hydrophile-lipophile balance) of the surfactant (C2) as mentioned herein is an index which expresses the ratio of the relative affinity of a surfactant for both liquids in an oil-water system, and can be determined by the Griffin method (J. Soc. Cosm. Chem., 1954, 5: 249-256) from the following formula, as with the component (B'):

HLB = 20 × [(molecular weight of hydrophilic group contained in surfactant)/(molecular weight of surfactant)]

Examples of the hydrophilic group contained in the surfactant include a hydroxy group and an ethyleneoxy group.

From the viewpoint of improving emulsion stability, the content of the surfactant (C2) in the emulsified cosmetic of the present invention is preferably 0.03 mass% or more, more preferably 0.05 mass% or more, and further more preferably 0.1 mass% or more, and is preferably 1 mass% or less, more preferably 0.7 mass% or less, further more preferably 0.5 mass% or less, and even more preferably 0.3 mass% or less. More specifically, the content of the surfactant (C2) in the emulsified cosmetic of the present invention is preferably from 0.03 to 1 mass%, more preferably from 0.05 to 0.7 mass%, further more preferably from 0.1 to 0.5 mass%, and even more preferably from 0.1 to 0.3 mass%, from the same viewpoint as above.

From the viewpoint of improving the adhesion of the component (A) to the skin surface and improving the UV protection effect, and from the viewpoint of improving the wear resistance and water resistance of the cosmetic coating film, the surface tension of the component (C) is less than 0.0728 N/m, preferably 0.0720 N/m or less, more preferably 0.0700 N/m or less, further more preferably 0.0600 N/m or less, even more preferably 0.0500 N/m or less, even more preferably 0.0450 N/m or less, even more preferably 0.0400 N/m or less, even more preferably 0.0370 N/m or less, and even more preferably 0.0350 N/m or less, and is preferably 0.0300 N/m or more, more preferably 0.0310 N/m or more, further more preferably 0.0320 N/m or more, and even more preferably 0.0330 N/m or more. More specifically, the surface tension of the component (C) is preferably from 0.0300 to 0.0720 N/m, more preferably from 0.0310 to 0.0700 N/m, further more preferably from 0.0320 to 0.0600 N/m, even more preferably from 0.0330 to 0.0500 N/m, even more preferably from 0.0330 to 0.0450 N/m, even more preferably from 0.0330 to 0.0400 N/m, even more preferably from 0.0330 to 0.0370 N/m, and even more preferably from 0.0330 to 0.0350 N/m, from the same viewpoints as above. The surface tension of the component (C) is measured by the method described in the Examples.

The content of the component (C) in the emulsified cosmetic of the present invention is 16 mass% or more, preferably 20 mass% or more, and more preferably 24 mass% or more, and is preferably 45 mass% or less, more preferably 40 mass% or less, and further more preferably 35 mass% or less, from the viewpoint of increasing the yield

value of the water-in-oil emulsified cosmetic, improving the adhesion of the component (A) to the skin surface, and improving the UV protection effect, and from the viewpoint of improving the wear resistance and water resistance of the cosmetic coating film. More specifically, the content of the component (C) in the emulsified cosmetic of the present invention is preferably from 16 to 45 mass%, more preferably from 20 to 40 mass%, and further more preferably from 24 to 35 mass%, from the same viewpoints as above. In the present invention, the component (C) is an aqueous-phase component other than the component (B); thus, the component (B) is not included in the calculation of the content of the component (C) in the emulsified cosmetic of the present invention. When the emulsified cosmetic of the present invention further contains a component (B'), the component (C) is an aqueous-phase component other than the component (B) and the component (B'); thus, the component (B') is also not included in the calculation of the content of the component (C) in the emulsified cosmetic of the present invention, as with the component (B). When the emulsified cosmetic of the present invention further contains a component (D) described later, the component (C) is an aqueous-phase component other than the component (B) and the component (D); thus, the component (D) is also not included in the calculation of the content of the component (C) in the emulsified cosmetic of the present invention, as with the component (B).

When the component (C) contains water, an alcohol (C1), and a surfactant (C2), the total content of water, the alcohol (C1), and the surfactant (C2) in the component (C) is preferably 60 mass% or more, more preferably 70 mass% or more, further more preferably 80 mass% or more, and even more preferably 90 mass% or more, and is preferably 100 mass% or less, from the viewpoint of obtaining good emulsion stability. More specifically, the content of water, the alcohol (C1), and the surfactant (C2) in the component (C) is preferably from 60 to 100 mass%, more preferably from 70 to 100 mass%, further more preferably from 80 to 100 mass%, and even more preferably from 90 to 100 mass%, from the same viewpoint as above.

<Component (D): One or more members selected from the group consisting of an amino acid or a salt thereof (D1), a sugar alcohol (D2), and a water-soluble polymer (D3)>

From the viewpoint of improving the UV protection effect, improving the wear resistance and water resistance of the cosmetic coating film, and enhancing the effect of improving the stratum corneum through continuous application, the emulsified cosmetic of the present invention preferably further contains, as a component (D), one or more members selected from the group consisting of an amino acid or a salt thereof (D1), a sugar alcohol having 4 or more carbon atoms (D2), and a water-soluble polymer (D3) (hereinafter also referred to as "the component (D)"), and more preferably contains an amino acid or a salt thereof (D1), a sugar alcohol having 4 or more carbon atoms (D2), and a water-soluble polymer (D3).

[Amino acid or salt thereof (D1)]

Examples of the amino acid or salt thereof include betaine (trimethylglycine), proline, hydroxyproline, arginine, lysine, serine, glycine, alanine, phenylalanine, tyrosine, β-alanine, threonine, glutamic acid, glutamine, asparagine, aspartic acid, cysteine, cystine, methionine, leucine, isoleucine, valine, tryptophan, histidine, and taurine, or salts thereof.

Among these, from the viewpoint of improving the UV protection effect, improving the wear resistance and water resistance of the cosmetic coating film, and enhancing the effect of improving the stratum corneum through continuous application, betaine (trimethylglycine) is preferred.

[Sugar alcohol having 4 or more carbon atoms (D2)]

Examples of the sugar alcohol having 4 or more carbon atoms include erythritol, xylitol, sorbitol, mannitol, maltitol, lactitol, and the like. Among these, from the viewpoint of improving the UV protection effect, improving the wear resistance and water resistance of the cosmetic coating film, and enhancing the effect of improving the stratum corneum through continuous application, xylitol is preferred.

[Water-soluble polymer (D3)]

The water-soluble polymer is not limited as long as it is used for general cosmetics. Any of natural, semi-synthetic, and synthetic polymers can be used.

Examples of natural polymers include xanthan gum, carrageenan, alginic acid, and the like.

Examples of semi-synthetic polymers include modified polysaccharides, such as hydroxycellulose, hydroxyethylcellulose, hydroxypropylcellulose, sodium carboxymethylcellulose, methylcellulose, hydroxymethylcellulose, and cationized cellulose.

Examples of synthetic polymers include vinyl polymers containing a structural unit derived from (meth)acrylic acid or a structural unit derived from a (meth)acrylic acid derivative, such as crosslinked polyacrylic acid (carbomer, carboxyvinyl polymer), polyacrylic acid, (alkyl acrylate/methacrylate) copolymers and salts thereof, and polyacrylic acid

amide; polyvinylpyrrolidone, polyvinyl alcohol, cationized polyvinylpyrrolidone, and the like.

Among these, from the viewpoint of improving the UV protection effect, improving the wear resistance and water resistance of the cosmetic coating film, and enhancing the effect of improving the stratum corneum through continuous application, xanthan gum is preferred.

As described above, the emulsified cosmetic of the present invention preferably contains, as the component (D), one or more members selected from the group consisting of trimethylglycine (hereinafter also referred to as "the component (d1)"), xylitol (hereinafter also referred to as "the component (d2)"), and xanthan gum (hereinafter also referred to as "the component (d3)"); more preferably at least two members selected from the group consisting of trimethylglycine (component (d1)), xylitol (component (d2)), and xanthan gum (component (d3)); and further more preferably trimethylglycine (component (d1)), xylitol (component (d2)), and xanthan gum (component (d3)).

The content of the component (D) in the emulsified cosmetic of the present invention is preferably 0.05 mass% or more, more preferably 0.10 mass% or more, and further more preferably 0.15 mass% or more, and is preferably 3.0 mass% or less, more preferably 2.0 mass% or less, further more preferably 1.0 mass% or less, and even more preferably 0.50 mass% or less, from the viewpoint of improving the UV protection effect, improving the wear resistance and water resistance of the cosmetic coating film, and enhancing the effect of improving the stratum corneum through continuous application. More specifically, the content of the component (D) in the emulsified cosmetic of the present invention is preferably from 0.05 to 3.0 mass%, more preferably from 0.10 to 2.0 mass%, further more preferably from 0.15 to 1.0 mass%, and even more preferably from 0.15 to 0.50 mass%, from the same viewpoint as above.

When an amino acid or a salt thereof (D1), a sugar alcohol having 4 or more carbon atoms (D2), and a water-soluble polymer (D3) are contained as the component (D), the mass ratio of the content of the water-soluble polymer (component (D3)) to the total content of the amino acid or salt thereof (component (D1)) and the sugar alcohol having 4 or more carbon atoms (component (D2)) in the emulsified cosmetic of the present invention [component (D3)/[component (D1) + component (D2)]] is preferably 0.005 or more, more preferably 0.010 or more, and further more preferably 0.013 or more, and is preferably 0.10 or less, more preferably 0.070 or less, further more preferably 0.050 or less, and even more preferably 0.030 or less, from the viewpoint of improving the UV protection effect, improving the wear resistance and water resistance of the cosmetic coating film, and enhancing the effect of improving the stratum corneum through continuous application. More specifically, the mass ratio [component (D3)/[component (D1) + component (D2)]] is preferably from 0.005 to 0.10, more preferably from 0.010 to 0.070, further more preferably from 0.013 to 0.050, and even more preferably from 0.013 to 0.030, from the same viewpoint as above.

When the component (D) is preferably a combination of trimethylglycine (component (d1)), xylitol (component (d2)), and xanthan gum (component (d3)), the mass ratio of the content of xanthan gum (component (d3)) to the total content of trimethylglycine (component (d1)) and xylitol (component (d2)) in the emulsified cosmetic of the present invention [component (d3)/[component (d1) + component (d2)]] is preferably 0.005 or more, more preferably 0.010 or more, and further more preferably 0.013 or more, and is preferably 0.10 or less, more preferably 0.070 or less, further more preferably 0.050 or less, and even more preferably 0.030 or less, from the viewpoint of improving the UV protection effect, improving the wear resistance and water resistance of the cosmetic coating film, and enhancing the effect of improving the stratum corneum through continuous application. More specifically, the mass ratio [component (d3)/[component (d1) + component (d2)]] is preferably from 0.005 to 0.10, more preferably from 0.010 to 0.070, further more preferably from 0.013 to 0.050, and even more preferably from 0.013 to 0.030, from the same viewpoint as above.

<Component (E): Oil agent which is liquid at 25°C>

The emulsified cosmetic of the present invention further contains, as a component (E), an oil agent which is liquid at 25°C (hereinafter also referred to as "the component (E)"), from the viewpoint of improving emulsion stability, from the viewpoint of improving the UV protection effect, and from the viewpoint of improving the wear resistance and water resistance of the cosmetic coating film. In the present invention, the definition of "solid" is as described above.

The component (E) is not particularly limited as long as it is liquid at 25°C, and examples thereof include oil agents used for general cosmetics. The component (E) is preferably one or more members selected from the group consisting of a silicone oil, a hydrocarbon oil, an ester oil, and a higher fatty acid. Among these, the component (E) is more preferably one or more members selected from the group consisting of a silicone oil and a hydrocarbon oil, and further more preferably a silicone oil (E1) (hereinafter also referred to as "the component (E1)"). When the component (E) contains the component (E1), the component (A1) can be stably dispersed, the UV protection effect can be improved, and the wear resistance and water resistance of the cosmetic coating film can be improved.

The components (E) can be used singly or in combination of two or more.

[Silicone oil (E1)]

The silicone oil (E1) is preferably at least one member selected from the group consisting of dimethyl silicone and

modified silicone, more preferably dimethyl silicone, further more preferably dimethylpolysiloxane having a kinematic viscosity at 25°C of 1 $mm^2/s$ or more and 20 $mm^2/s$ or less, even more preferably dimethylpolysiloxane having a kinematic viscosity at 25°C of 1 $mm^2/s$ or more and 20 $mm^2/s$ or less.

Examples of the modified silicone include alkyl-modified silicone, phenyl-modified silicone, and the like.

The kinematic viscosity at 25°C of the silicone oil (E1) can be measured in accordance with ASTM D 445-46T or JIS Z 8803: 2011 using an Ubbelohde viscometer.

Commercial products of the silicone oil (E1) include "KF-96A-2cs" (dimethylpolysiloxane), "KF-96A-6cs" (dimethylpolysiloxane), "KF-96A-10cs" (dimethylpolysiloxane), and "KF-96A-20cs" (dimethylpolysiloxane), manufactured by Shin-Etsu Chemical Co., Ltd.

The hydrocarbon oil is preferably one or more members selected from the group consisting of liquid paraffin, hydrogenated polyisobutene (light isoparaffin, light liquid isoparaffin, liquid isoparaffin, and heavy liquid isoparaffin), cycloparaffin, liquid ozokerite, squalene, squalane, pristane, α-olefin oligomer, polybutene, and isohexadecane. Among these, the hydrocarbon oil is more preferably hydrogenated polyisobutene, and further more preferably liquid isoparaffin.

Commercial products of the hydrocarbon oil include "Parleam EX" (liquid isoparaffin) manufactured by NOF Corporation.

Examples of the ester oil include synthetic ester oils, natural fats/oils, and the like.

Specific examples of the ester oil include cetyl 2-ethylhexanoate, isononyl isononanoate, isotridecyl isononanoate, isopropyl myristate, isopropyl palmitate, 2-ethylhexyl palmitate, 2-ethylhexyl stearate, stearyl stearate, alkyl benzoate (C12-15), caprylic/capric triglyceride, glyceryl tri(2-ethylhexanoate), neopentyl glycol dicaprate, and neopentyl glycol diethylhexanoate.

Examples of the higher fatty acid include fatty acids having 12 or more and 22 or less carbon atoms, such as oleic acid and isostearic acid.

The content of the component (E) in the emulsified cosmetic of the present invention is preferably 25 mass% or more, more preferably 27 mass% or more, and further more preferably 30 mass% or more, and is preferably 55 mass% or less, more preferably 50 mass% or less, and further more preferably 47 mass% or less, from the viewpoint of improving emulsion stability, from the viewpoint of improving the UV protection effect, and from the viewpoint of improving the wear resistance and water resistance of the cosmetic coating film. More specifically, the content of the component (E) in the emulsified cosmetic of the present invention is preferably from 25 to 55 mass%, more preferably from 27 to 50 mass%, and further more preferably from 30 to 47 mass%, from the same viewpoints as above.

When the emulsified cosmetic of the present invention contains a silicone oil (E1) as the component (E), the content of the component (E1) in the total amount of the component (E) in the emulsified cosmetic of the present invention is preferably 30 mass% or more, more preferably 35 mass% or more, and further more preferably 40 mass% or more, and is preferably 85 mass% or less, more preferably 80 mass% or less, further more preferably 75 mass% or less, and even more preferably 70 mass% or less, from the viewpoint of stably dispersing the component (A1) and improving the UV protection effect, and from the viewpoint of improving the wear resistance and water resistance of the cosmetic coating film. More specifically, the content of the component (E1) in the total amount of the component (E) in the emulsified cosmetic of the present invention is preferably from 30 to 85 mass%, more preferably from 35 to 80 mass%, further more preferably from 40 to 75 mass%, and even more preferably from 40 to 70 mass%, from the same viewpoints as above.

When the emulsified cosmetic of the present invention contains a silicone oil (E1) as the component (E), the mass ratio of the content of the component (E1) to the content of the component (A1) in the emulsified cosmetic of the present invention [component (E1)/component (A1)] is preferably 0.80 or more, more preferably 0.85 or more, further more preferably 0.90 or more, even more preferably 0.95 or more, and even more preferably 1.0 or more, from the viewpoint of stably dispersing the component (A1) and improving the UV protection effect, from the viewpoint of improving the wear resistance and water resistance of the cosmetic coating film, and from the viewpoint of the effect of improving the stratum corneum through continuous application, and is preferably 5.0 or less, more preferably 4.5 or less, further more preferably 3.0 or less, even more preferably 2.0 or less, even more preferably 1.7 or less, even more preferably 1.5 or less, and even more preferably 1.3 or less, from the viewpoint of improving the UV protection effect. More specifically, the mass ratio [component (E1)/component (A1)] is preferably from 0.80 to 5.0, more preferably from 0.85 to 5.0, further more preferably from 0.90 to 4.5, even more preferably from 0.95 to 3.0, even more preferably from 0.95 to 2.0, even more preferably from 1.0 to 1.7, even more preferably from 1.0 to 1.5, and even more preferably from 1.0 to 1.3, from the same viewpoints as above.

In addition to the components (A) to (E), the emulsified cosmetic of the present invention may appropriately contain optional components, which are used depending on the purpose of cosmetics, within a range which does not impair the effects of the present invention. Such optional components include UV absorbers, neutralizing agents, pH adjusters, disinfectants, anti-inflammatory agents, preservatives, colorants, chelating agents, skin whitening agents, antiperspirants, insect repellents, bioactive components, salts, antioxidants, and fragrances, other than the components (A) to (E).

<Component (F): UV absorber>

From the viewpoint of further improving the UV protection effect, the emulsified cosmetic of the present invention may contain one or more UV absorbers selected from the group consisting of liquid organic UV absorbers and solid organic UV absorbers (hereinafter also referred to as "the component (F)"). In the present invention, the definitions of "liquid" and "solid" are as described above.

Examples of liquid organic UV absorbers include cinnamate-based UV absorbers, such as 2-ethylhexyl p-methoxycinnamate, 2-ethoxyethyl p-methoxycinnamate, isopropyl p-methoxycinnamate and diisopropyl cinnamate ester mixture, and methylbis(trimethylsiloxy)silylisopentyl trimethoxycinnamate; p-aminobenzoate-based UV absorbers, such as amyl p-dimethylaminobenzoate and 2-ethylhexyl p-dimethylaminobenzoate; salicylate-based UV absorbers, such as ethylene glycol salicylate, 2-ethylhexyl salicylate, butyloctyl salicylate, benzyl salicylate, and homomenthyl salicylate; octocrylene; dimethicodiethyl benzalmalonate; a copolymer of adipic acid and neopentyl glycol end-capped with octyldodecanol or cyanodiphenylpropenoic acid, and the like. These liquid organic UV absorbers can be contained singly or in combination of two or more.

Examples of commercially available liquid organic UV absorbers include "UVINUL MC80" (2-ethylhexyl p-methoxycinnamate) (manufactured by BASF); "PARSOL 340" (octocrylene) (manufactured by DSM Nutrition Japan K.K.); "PARSOL EHS" (2-ethylhexyl salicylate), "PARSOL HMS" (homomenthyl salicylate), and "PARSOL SLX" (Polysilicone-15 (INCI Name: Polysilicone-15) (dimethicodiethyl benzalmalonate)) (all manufactured by DSM); and "Polycrylene" (Polyester-8 (INCI Name: Polyester-8) (a copolymer of adipic acid and neopentyl glycol end-capped with octyldodecanol or cyanodiphenylpropenoic acid)) (manufactured by Hallstar).

Examples of solid organic UV absorbers include 4-tert-butyl-4'-methoxydibenzoylmethane, hexyl 2-(4-diethylamino-2-hydroxybenzoyl)benzoate, 2,4,6-tris[4-(2-ethylhexyloxycarbonyl)anilino]-1,3,5-triazine, 2-hydroxy-4-methoxybenzophenone, drometrizole trisiloxane, 2,4-bis{[4-(2-ethylhexyloxy)-2-hydroxy]phenyl}-6-(4-methoxyphenyl)-1,3,5-triazine, 2-ethylhexyl dimethoxybenzylidene dioxoimidazolidine propionate, and the like. These solid organic UV absorbers can be contained singly or in combination of two or more.

Examples of commercially available solid organic UV absorbers include "PARSOL 1789" (4-tert-butyl-4'-methoxydibenzoylmethane) (manufactured by DSM); "UVINUL A PLUS" (hexyl 2-(4-diethylamino-2-hydroxybenzoyl) benzoate), "UVINUL T-150" (2,4,6-tris[4-(2-ethylhexyloxycarbonyl)anilino]-1,3,5-triazine), "UVINUL M40" (2-hydroxy-4-methoxybenzophenone), and "TINOSORB S" (2,4-bis{[4-(2-ethylhexyloxy)-2-hydroxy]phenyl}-6-(4-methoxyphenyl)-1,3,5-triazine) (all manufactured by BASF); "Mosacare A440" (drometrizole trisiloxane) (manufactured by UFC Corporation); "Soft Shade DH" (2-ethylhexyl dimethoxybenzylidene dioxoimidazolidine propionate) (manufactured by Ajinomoto Co., Inc.), and the like.

The content of the component (F) in the emulsified cosmetic of the present invention may be preferably 3 mass% or less, more preferably 2 mass% or less, further more preferably 1 mass% or less, even more preferably 0.5 mass% or less, even more preferably 0.3 mass% or less, and even more preferably 0 mass%. More specifically, the content of the component (F) in the emulsified cosmetic of the present invention is preferably from 0 to 3 mass%, more preferably from 0 to 2 mass%, further more preferably from 0 to 1 mass%, even more preferably from 0 to 0.5 mass%, and even more preferably from 0 to 0.3 mass%.

The yield value of the emulsified cosmetic of the present invention is preferably 0.1 Pa or more, more preferably 0.3 Pa or more, further more preferably 0.5 Pa or more, and even more preferably 0.7 Pa or more, from the viewpoint of improving the adhesion of the component (A) to the skin surface and improving the UV protection effect, and from the viewpoint of improving the wear resistance and water resistance of the cosmetic coating film, and is preferably 30 Pa or less, more preferably 20 Pa or less, further more preferably 10 Pa or less, even more preferably 5 Pa or less, even more preferably 3 Pa or less, even more preferably 1 Pa or less, and even more preferably 0.8 Pa or less, from the viewpoint of the effect of improving the stratum corneum through continuous application. More specifically, the yield value of the emulsified cosmetic of the present invention is preferably from 0.1 to 30 Pa, more preferably from 0.3 to 20 Pa, further more preferably from 0.3 to 10 Pa, even more preferably from 0.3 to 5 Pa, even more preferably from 0.3 to 3 Pa, even more preferably from 0.3 to 1 Pa, even more preferably from 0.5 to 1 Pa, and even more preferably from 0.7 to 0.8 Pa, from the same viewpoints as above. The yield value of the emulsified cosmetic of the present invention is measured by the method described in the Examples.

The emulsified cosmetic of the present invention can be suitably used as skin cosmetics, such as sunscreen cosmetics, facial cleansers, cleansing cosmetics, packs, and massage cosmetics; and hair cosmetics, such as shampoos, rinses, and conditioners. Among these, the water-in-oil emulsified cosmetic of the present invention has an excellent UV protection effect, and is thus preferably applied to sunscreen cosmetics (e.g., lotions, creams, emulsions, and serums), suntans, cosmetic bases, foundations, and the like for sunscreen use.

The emulsified cosmetic of the present invention can be in the form of an emulsion, cream, gel, paste, solid, multilayer, or the like, and can also be used as a sheet, spray, or mousse.

The emulsified cosmetic of the present invention contains the component (A) uniformly emulsified and dispersed

therein and has an excellent UV protection effect, and its cosmetic coating film has excellent wear resistance and water resistance. Therefore, the emulsified cosmetic of the present invention is particularly useful as a sunscreen cosmetic.

(Method for producing water-in-oil emulsified cosmetic)

The method for producing the water-in-oil emulsified cosmetic of the present invention is not particularly limited, and any known method can be appropriately used depending on the form of the water-in-oil emulsified cosmetic. For example, there is a method containing blending components (A), (B), and (C), as well as the optional components mentioned above as necessary, and mixing them uniformly with a homomixer or the like. Among such methods, from the viewpoint of stably dispersing the component (A) and improving the UV protection effect, and from the viewpoint of improving the wear resistance and water resistance of the cosmetic coating film, the method for producing the emulsified cosmetic of the present invention is preferably a method containing dispersing the component (A), while heating and dissolving an oily composition containing the component (B) and the oil-soluble optional component mentioned above as necessary, to obtain a dispersion, and then mixing and emulsifying the cooled dispersion with an aqueous composition containing the component (C) and the water-soluble optional component mentioned above as necessary. More specifically, a method containing the following steps I and II is more preferred.

In the present invention, the "oil-soluble component" as mentioned herein refers to a component which has a non-uniform appearance when visually observed in a mixture obtained by mixing 100 parts by mass of water and 3 parts by mass of the component at 25°C. The phrase "the mixture has a non-uniform appearance" as mentioned herein means that separation is visually observed.

Step I: a step of heating and dissolving the component (B) in the component (E), and then adding and dispersing the component (A) to obtain a dispersion

Step II: a step of adding and mixing the component (C) and optionally the component (D) with the dispersion obtained in step I, followed by emulsification

In step I, the heating and dissolution of the component (B) in the component (E) and the addition of the component (A) are each preferably carried out with stirring at a temperature in the range of 40°C or higher and 90°C or lower.

In step II, it is preferable to add and mix the component (C) and optionally the component (D), while stirring the dispersion obtained in step I preferably kept at 40°C or higher and 90°C or lower, followed by emulsification.

Regarding the above embodiments, the present invention further discloses the following embodiments.

<1> A water-in-oil emulsified cosmetic comprising the following components (A), (B), and (C):

(A) an UV scattering agent having a hydrophobicity degree of 73% or less in accordance with methanol titration;
(B) an amphiphilic substance which is solid at 25°C; and
(C) an aqueous-phase component (provided that the component (B) is excluded),

the component (A) comprising dimethicone-treated zinc oxide microparticles (A1),
a mass ratio of a content of the component (A1) to a content of the component (A) [component (A1)/component (A)] being 0.38 or more,
the component (C) having a surface tension of less than 0.0728 N/m, and
a content of the component (C) being 16 mass% or more.

<2> The water-in-oil emulsified cosmetic according to <1>, wherein the hydrophobicity degree of the component (A) in accordance with methanol titration is preferably 70% or less, more preferably 65% or less, and further more preferably 60% or less, and is preferably 30% or more, more preferably 35% or more, further more preferably 40% or more, and even more preferably 45% or more.

<3> The water-in-oil emulsified cosmetic according to <1>, wherein the hydrophobicity degree of the component (A) in accordance with methanol titration is preferably from 30 to 70%, more preferably from 35 to 65%, further more preferably from 40 to 60%, and even more preferably from 45 to 60%.

<4> The water-in-oil emulsified cosmetic according to any one of <1> to <3>, wherein the component (A) further comprises hydrophobically-treated titanium oxide microparticles (A2).

<5> The water-in-oil emulsified cosmetic according to <4>, wherein the hydrophobization treatment of the component (A2) is preferably one or more members selected from the group consisting of silicone treatment, alkylalkoxysilane treatment, and fatty acid treatment; more preferably one or more members selected from the group consisting of silicone treatment using, as a surface treatment agent, one or more members selected from the group consisting of methylpolysiloxane, dimethylpolysiloxane (dimethicone), methylhydrogenpolysiloxane, and a dimethylsiloxane/-

methylhydrogensiloxane copolymer (hydrogen dimethicone), alkylalkoxysilane treatment using, as a surface treatment agent, an alkylalkoxysilane having a linear or branched alkyl group having 6 or more and 20 or less carbon atoms, and fatty acid treatment using, as a surface treatment agent, a linear or branched higher fatty acid having 14 or more and 22 or less carbon atoms; and further more preferably fatty acid treatment using, as a surface treatment agent, a linear or branched higher fatty acid having 14 or more and 22 or less carbon atoms.

<6> The water-in-oil emulsified cosmetic according to any one of <1> to <5>, wherein the component (A) has an average primary particle size of preferably 3 nm or more, more preferably 5 nm or more, and further more preferably 10 nm or more, and of preferably 800 nm or less, more preferably 700 nm or less, further more preferably 500 nm or less, even more preferably 300 nm or less, even more preferably 100 nm or less, even more preferably 50 nm or less, and even more preferably 40 nm or less.

<7> The water-in-oil emulsified cosmetic according to any one of <1> to <5>, wherein the component (A) has an average primary particle size of preferably from 3 nm to 800 nm, more preferably from 5 to 700 nm, further more preferably from 5 to 500 nm, even more preferably from 5 to 300 nm, even more preferably from 5 to 100 nm, even more preferably from 5 to 50 nm, even more preferably from 10 to 50 nm, and even more preferably from 10 to 40 nm.

<8> The water-in-oil emulsified cosmetic according to any one of <1> to <7>, wherein the content of the component (A) is preferably 5 mass% or more, more preferably 10 mass% or more, further more preferably 15 mass% or more, even more preferably 20 mass% or more, and even more preferably 25 mass% or more, and is preferably 40 mass% or less, more preferably 37 mass% or less, further more preferably 35 mass% or less, and even more preferably 30 mass% or less.

<9> The water-in-oil emulsified cosmetic according to any one of <1> to <7>, wherein the content of the component (A) is preferably from 5 to 40 mass%, more preferably from 10 to 37 mass%, further more preferably from 15 to 35 mass%, even more preferably from 20 to 35 mass%, even more preferably from 25 to 35 mass%, and even more preferably from 25 to 30 mass%.

<10> The water-in-oil emulsified cosmetic according to any one of <1> to <9>, wherein the mass ratio of the content of the component (A1) to the content of the component (A) [component (A1)/component (A)] is preferably 0.50 or more, more preferably 0.60 or more, further more preferably 0.63 or more, and even more preferably 0.65 or more, and is preferably 1.0 or less, more preferably 0.80 or less, further more preferably 0.76 or less, even more preferably 0.72 or less, and even more preferably 0.70 or less.

<11> The water-in-oil emulsified cosmetic according to any one of <1> to <9>, wherein the mass ratio of the content of the component (A1) to the content of the component (A) [component (A1)/component (A2)] is preferably from 0.38 to 1.0, more preferably from 0.38 to 0.80, further more preferably from 0.38 to 0.76, even more preferably from 0.50 to 0.76, even more preferably from 0.60 to 0.76, even more preferably from 0.63 to 0.76, even more preferably from 0.63 to 0.72, and even more preferably from 0.65 to 0.70.

<12> The water-in-oil emulsified cosmetic according to any one of <1> to <11>, wherein when the component (A) further comprises hydrophobically-treated titanium oxide microparticles (A2), a mass ratio of a content of the dimethicone-treated zinc oxide microparticles (A1) to a content of the hydrophobically-treated titanium oxide microparticles (A2) [dimethicone-treated zinc oxide microparticles (A1)/hydrophobically-treated titanium oxide microparticles (A2)] is preferably 0.50 or more, more preferably 1.0 or more, further more preferably 1.5 or more, and even more preferably 2.0 or more, and is preferably 3.5 or less, more preferably 3.4 or less, further more preferably 3.3 or less, and even more preferably 3.2 or less.

<13> The water-in-oil emulsified cosmetic according to any one of <1> to <11>, wherein when the component (A) further comprises hydrophobically-treated titanium oxide microparticles (A2), the mass ratio of the content of the dimethicone-treated zinc oxide microparticles (A1) to the content of the hydrophobically-treated titanium oxide microparticles (A2) [dimethicone-treated zinc oxide microparticles/hydrophobically-treated titanium oxide microparticles (A2)] is preferably from 0.50 to 3.5, more preferably from 1.0 to 3.4, further more preferably from 1.5 to 3.3, and even more preferably from 2.0 to 3.2.

<14> The water-in-oil emulsified cosmetic according to any one of <1> to <13>, wherein when the component (A) further comprises hydrophobically-treated titanium oxide microparticles (A2), a content of the component (A2) is preferably less than 10 mass%, more preferably 9 mass% or less, and further more preferably 8 mass% or less, and is preferably 1 mass% or more, more preferably 3 mass% or more, and further more preferably 5 mass% or more.

<15> The water-in-oil emulsified cosmetic according to any one of <1> to <13>, wherein the content of the component (A2) is preferably from 1 to 9 mass%, more preferably from 3 to 9 mass%, further more preferably from 5 to 9 mass%, and even more preferably from 5 to 8 mass%.

<16> The water-in-oil emulsified cosmetic according to any one of <1> to <15>, wherein the component (B) is preferably one or more members selected from the group consisting of ceramide, an alcohol having 8 or more and 22 or less carbon atoms, a dextrin fatty acid ester having 8 or more and 22 or less carbon atoms, a glycerin fatty acid ester having 8 or more and 22 or less carbon atoms, an alkyl glyceryl ether having an alkyl group having 8 or more and 22 or less carbon atoms, a sorbitan fatty acid ester having 8 or more and 22 or less carbon atoms, and a polyoxyethylene

sorbitan fatty acid ester having 8 or more and 22 or less carbon atoms; more preferably one or more members selected from the group consisting of ceramide and a dextrin fatty acid ester having 8 or more and 22 or less carbon atoms; further more preferably a dextrin fatty acid ester having 8 or more and 22 or less carbon atoms; and even more preferably a combination of ceramide and a dextrin fatty acid ester having 8 or more and 22 or less carbon atoms.

<17> The water-in-oil emulsified cosmetic according to any one of <1> to <16>, wherein a content of the component (B) is preferably 0.1 mass% or more, more preferably 0.5 mass% or more, further more preferably 1 mass% or more, even more preferably 2 mass% or more, and even more preferably 3 mass% or more, and is preferably 12 mass% or less, more preferably 10 mass% or less, and further more preferably 9 mass% or less.

<18> The water-in-oil emulsified cosmetic according to any one of <1> to <16>, wherein the content of the component (B) is preferably from 0.1 to 12 mass%, more preferably from 0.5 to 12 mass%, further more preferably from 1 to 10 mass%, even more preferably from 2 to 10 mass%, even more preferably from 2 to 9 mass%, and even more preferably from 3 to 9 mass%.

<19> The water-in-oil emulsified cosmetic according to any one of <1> to <18>, wherein when ceramide and a dextrin fatty acid ester having 8 or more and 22 or less carbon atoms are used in combination as the component (B), a mass ratio of a content of the ceramide (component (b1)) to a content of the dextrin fatty acid ester (component (b2)) [component (b1)/component (b2)] is preferably 0.5 or more, more preferably 1 or more, further more preferably 3 or more, even more preferably 5 or more, and even more preferably 7 or more, and is preferably 30 or less, more preferably 20 or less, further more preferably 15 or less, and even more preferably 13 or less.

<20> The water-in-oil emulsified cosmetic according to any one of <1> to <18>, wherein when ceramide and a dextrin fatty acid ester having 8 or more and 22 or less carbon atoms are used in combination as the component (B), the mass ratio of the content of the ceramide (component (b1)) to the content of the dextrin fatty acid ester (component (b2)) [component (b1)/component (b2)] is preferably from 0.5 to 30, more preferably from 1 to 20, further more preferably from 3 to 15, even more preferably from 5 to 13, and even more preferably from 7 to 13.

<21> The water-in-oil emulsified cosmetic according to any one of <1> to <20>, wherein a mass ratio of the content of the component (A) to the content of the component (B) [component (A)/component (B)] is preferably 1 or more, more preferably 2 or more, and further more preferably 3 or more, and is preferably 100 or less, more preferably 70 or less, further more preferably 50 or less, even more preferably 30 or less, even more preferably 25 or less, even more preferably 20 or less, even more preferably 15 or less, and even more preferably 12 or less.

<22> The water-in-oil emulsified cosmetic according to any one of <1> to <20>, wherein the mass ratio of the content of the component (A) to the content of the component (B) [component (A)/component (B)] is preferably from 1 to 100, more preferably from 1 to 70, further more preferably from 1 to 50, even more preferably from 1 to 30, even more preferably from 2 to 25, even more preferably from 3 to 20, even more preferably from 3 to 15, and even more preferably from 3 to 12.

<23> The water-in-oil emulsified cosmetic according to any one of <1> to <22>, further comprising, as a component (B'), an amphiphilic substance which is liquid at 25°C.

<24> The water-in-oil emulsified cosmetic according to any one of <23>, wherein the component (B') is preferably an oil-soluble surfactant having an HLB of 14.5 or less; more preferably one or more members selected from the group consisting of polyether-modified silicone, polyglycerin-modified silicone, acrylic silicone, a sorbitan fatty acid ester, a glycerin fatty acid ester, and a polyoxyethylene hydrogenated castor oil; further more preferably one or more members selected from the group consisting of polyether-modified silicone, polyglycerin-modified silicone, and acrylic silicone; and even more preferably polyether-modified silicone.

<25> The water-in-oil emulsified cosmetic according to <23> or <24>, wherein a content of the component (B') is preferably 0.5 mass% or more, more preferably 1 mass% or more, and further more preferably 2 mass% or more, and is preferably 5 mass% or less, more preferably 4 mass% or less, and further more preferably 3 mass% or less.

<26> The water-in-oil emulsified cosmetic according to <23> or <24>, wherein the content of the component (B') is preferably from 0.5 to 5 mass%, more preferably from 1 to 4 mass%, and further more preferably from 2 to 3 mass%.

<27> The water-in-oil emulsified cosmetic according to any one of <23> to <26>, wherein a mass ratio of the content of the component (B') to the content of the component (B) [component (B')/component (B)] is preferably 0.10 or more, more preferably 0.20 or more, and further more preferably 0.30 or more, and is preferably 12 or less, more preferably 8 or less, further more preferably 6 or less, even more preferably 4 or less, even more preferably 2 or less, and even more preferably 1 or less.

<28> The water-in-oil emulsified cosmetic according to any one of <23> to <26>, wherein the mass ratio of the content of the component (B') to the content of the component (B) [component (B')/component (B)] is preferably from 0.10 to 12, more preferably from 0.20 to 8, further more preferably from 0.30 to 6, even more preferably from 0.30 to 4, even more preferably from 0.30 to 2, and even more preferably from 0.30 to 1.

<29> The water-in-oil emulsified cosmetic according to any one of <1> to <28>, wherein when the emulsified cosmetic further comprises an amphiphilic substance which is liquid at 25°C as the component (B') and a dextrin fatty acid ester having 8 or more and 22 or less carbon atoms (b2) as the component (B), a mass ratio of the content of the component

(B') to a content of the component (b2) [component (B')/component (b2)] is preferably 3 or more, more preferably 4 or more, further more preferably 6 or more, and even more preferably 8 or more, and is preferably 12 or less, more preferably 11 or less, and further more preferably 10 or less.

<30> The water-in-oil emulsified cosmetic according to any one of <1> to <28>, wherein when the emulsified cosmetic further comprises an amphiphilic substance which is liquid at 25°C as the component (B') and a dextrin fatty acid ester having 8 or more and 22 or less carbon atoms (b2) as the component (B), the mass ratio of the content of the component (B') to the content of the component (b2) [component (B')/component (b2)] is preferably from 3 to 12, more preferably from 3 to 11, further more preferably from 4 to 11, even more preferably from 6 to 10, and even more preferably from 8 to 10.

<31> The water-in-oil emulsified cosmetic according to any one of <1> to <30>, wherein the component (C) preferably comprises one or more members selected from the group consisting of water, an alcohol (C1), and a surfactant (provided that the component (B) and the component (B') are excluded) (C2); and more preferably water, an alcohol (C1), and a surfactant (provided that the component (B) and the component (B') are excluded) (C2).

<32> The water-in-oil emulsified cosmetic according to <31>, wherein the alcohol (C1) is preferably one or more members selected from the group consisting of monohydric alcohols and polyhydric alcohols; more preferably one or more members selected from the group consisting of saturated monohydric alcohols having 2 or more and 4 or less carbon atoms and polyhydric alcohols having 2 or more and 12 or less carbon atoms; further more preferably one or more members selected from the group consisting of ethanol, ethylene glycol, diethylene glycol, 1,2-propanediol (propylene glycol), 1,3-propanediol (propanediol), dipropylene glycol, 1,3-butanediol (1,3-butylene glycol), and glycerin; even more preferably one or more members selected from the group consisting of ethanol, 1,3-propanediol, dipropylene glycol, 1,3-butanediol, and glycerin; even more preferably one or more members selected from the group consisting of ethanol, dipropylene glycol, 1,3-butanediol, and glycerin; and even more preferably one or more members selected from the group consisting of dipropylene glycol, 1,3-butanediol, and glycerin.

<33> The water-in-oil emulsified cosmetic according to <31> or <32>, wherein the surfactant (C2) is preferably a nonionic surfactant; more preferably one or more members selected from the group consisting of a polyoxyalkylene fatty acid ester, a polyoxyalkylene alkyl ether, a polyoxyalkylene alkenyl ether, an alkyl glucoside, a sucrose fatty acid ester, a polyglycerin fatty acid ester, a polyoxyalkylene hydrogenated castor oil, an alkyl saccharide, an alkylamine oxide, and an alkyl amidoamine oxide; further more preferably a polyoxyalkylene alkyl ether; and even more preferably a polyoxyalkylene alkyl ether having an alkyl group having 8 or more and 22 or less carbon atoms.

<34> The water-in-oil emulsified cosmetic according to any one of <31> to <33>, wherein the surfactant (C2) has an HLB of preferably 8 or more, more preferably 10 or more, and further more preferably 12 or more, and of preferably 18 or less, more preferably 17 or less, and further more preferably 16 or less.

<35> The water-in-oil emulsified cosmetic according to any one of <31> to <33>, wherein the surfactant (C2) has an HLB of preferably from 8 to 18, more preferably from 10 to 17, and further more preferably from 12 to 16.

<36> The water-in-oil emulsified cosmetic according to any one of <31> to <35>, wherein a content of the alcohol (C1) is preferably 3 mass% or more, more preferably 5 mass% or more, and further more preferably 7 mass% or more, and is preferably 40 mass% or less, more preferably 35 mass% or less, and further more preferably 30 mass% or less.

<37> The water-in-oil emulsified cosmetic according to any one of <31> to <35>, wherein the content of the alcohol (C1) is preferably from 3 to 40 mass%, more preferably from 5 to 35 mass%, and further more preferably from 7 to 30 mass%.

<38> The water-in-oil emulsified cosmetic according to any one of <31> to <37>, wherein a content of the surfactant (C2) is preferably 0.03 mass% or more, more preferably 0.05 mass% or more, and further more preferably 0.1 mass% or more, and is preferably 1 mass% or less, more preferably 0.7 mass% or less, further more preferably 0.5 mass% or less, and even more preferably 0.3 mass% or less.

<39> The water-in-oil emulsified cosmetic according to any one of <31> to <37>, wherein the content of the surfactant (C2) is preferably from 0.03 to 1 mass%, more preferably from 0.05 to 0.7 mass%, further more preferably from 0.1 to 0.5 mass%, and even more preferably from 0.1 to 0.3 mass%.

<40> The water-in-oil emulsified cosmetic according to any one of <1> to <39>, wherein the component (C) has a surface tension of preferably 0.0720 N/m or less, more preferably 0.0700 N/m or less, further more preferably 0.0600 N/m or less, even more preferably 0.0500 N/m or less, even more preferably 0.0450 N/m or less, even more preferably 0.0400 N/m or less, even more preferably 0.0370 N/m or less, and even more preferably 0.0350 N/m or less, and of preferably 0.0300 N/m or more, more preferably 0.0310 N/m or more, further more preferably 0.0320 N/m or more, and even more preferably 0.0330 N/m or more.

<41> The water-in-oil emulsified cosmetic according to any one of <1> to <39>, wherein the component (C) has a surface tension of preferably from 0.0300 to 0.0720 N/m, more preferably from 0.0310 to 0.0700 N/m, further more preferably from 0.0320 to 0.0600 N/m, even more preferably from 0.0330 to 0.0500 N/m, even more preferably from 0.0330 to 0.0450 N/m, even more preferably from 0.0330 to 0.0400 N/m, even more preferably from 0.0330 to 0.0370 N/m, and even more preferably from 0.0330 to 0.0350 N/m.

<42> The water-in-oil emulsified cosmetic according to any one of <1> to <41>, wherein the content of the component (C) is preferably 20 mass% or more, and more preferably 24 mass% or more, and is preferably 45 mass% or less, more preferably 40 mass% or less, and further more preferably 35 mass% or less.

<43> The water-in-oil emulsified cosmetic according to any one of <1> to <41>, wherein the content of the component (C) is preferably from 16 to 45 mass%, more preferably from 20 to 40 mass%, and further more preferably from 24 to 35 mass%.

<44> The water-in-oil emulsified cosmetic according to any one of <1> to <43>, wherein when the component (C) comprises water, an alcohol (C1), and a surfactant (C2), a total content of water, the alcohol (C1), and the surfactant (C2) in the component (C) is preferably 60 mass% or more, more preferably 70 mass% or more, further more preferably 80 mass% or more, and even more preferably 90 mass% or more, and is preferably 100 mass% or less.

<45> The water-in-oil emulsified cosmetic according to any one of <1> to <43>, wherein when the component (C) comprises water, an alcohol (C1), and a surfactant (C2), the content of water, the alcohol (C1), and the surfactant (C2) in the component (C) is preferably from 60 to 100 mass%, more preferably from 70 to 100 mass%, further more preferably from 80 to 100 mass%, and even more preferably from 90 to 100 mass%.

<46> The water-in-oil emulsified cosmetic according to any one of <1> to <45>, further comprising, as a component (D), preferably one or more members selected from the group consisting of an amino acid or a salt thereof (D1), a sugar alcohol having 4 or more carbon atoms (D2), and a water-soluble polymer (D3); more preferably one or more members selected from the group consisting of trimethylglycine, xylitol, and xanthan gum; further more preferably at least two members selected from the group consisting of trimethylglycine, xylitol, and xanthan gum; and even more preferably trimethylglycine, xylitol, and xanthan gum.

<47> The water-in-oil emulsified cosmetic according to <46>, wherein a content of the component (D) is preferably 0.05 mass% or more, more preferably 0.10 mass% or more, and further more preferably 0.15 mass% or more, and is preferably 3.0 mass% or less, more preferably 2.0 mass% or less, further more preferably 1.0 mass% or less, and even more preferably 0.50 mass% or less.

<48> The water-in-oil emulsified cosmetic according to <46>, wherein the content of the component (D) is preferably from 0.05 to 3.0 mass%, more preferably from 0.10 to 2.0 mass%, further more preferably from 0.15 to 1.0 mass%, and even more preferably from 0.15 to 0.50 mass%.

<49> The water-in-oil emulsified cosmetic according to any one of <46> to <48>, wherein when an amino acid or a salt thereof (D1), a sugar alcohol having 4 or more carbon atoms (D2), and a water-soluble polymer (D3) are contained as the component (D), a mass ratio of a content of the water-soluble polymer (component (D3)) to a total content of the amino acid or salt thereof (component (D1)) and the sugar alcohol having 4 or more carbon atoms (component (D2)) [component (D3)/[component (D1) + component (D2)]] is preferably 0.005 or more, more preferably 0.010 or more, and further more preferably 0.013 or more, and is preferably 0.10 or less, more preferably 0.070 or less, further more preferably 0.050 or less, and even more preferably 0.030 or less.

<50> The water-in-oil emulsified cosmetic according to any one of <46> to <48>, wherein when an amino acid or a salt thereof (D1), a sugar alcohol having 4 or more carbon atoms (D2), and a water-soluble polymer (D3) are contained as the component (D), the mass ratio of the content of the water-soluble polymer (component (D3)) to the total content of the amino acid or salt thereof (component (D1)) and the sugar alcohol having 4 or more carbon atoms (component (D2)) [component (D3)/[component (D1) + component (D2)]] is preferably from 0.005 to 0.10, more preferably from 0.010 to 0.070, further more preferably from 0.013 to 0.050, and even more preferably from 0.013 to 0.030.

<51> The water-in-oil emulsified cosmetic according to any one of <46> to <50>, wherein when the component (D) is a combination of trimethylglycine, xylitol, and xanthan gum, a mass ratio of a content of xanthan gum (component (d3)) to a total content of trimethylglycine (component (d1)) and xylitol (component (d2)) [component (d3)/[component (d1) + component (d2)]] is preferably 0.005 or more, more preferably 0.010 or more, and further more preferably 0.013 or more, and is preferably 0.10 or less, more preferably 0.070 or less, further more preferably 0.050 or less, and even more preferably 0.030 or less.

<52> The water-in-oil emulsified cosmetic according to any one of <46> to <50>, wherein when the component (D) is a combination of trimethylglycine, xylitol, and xanthan gum, the mass ratio of the content of xanthan gum (component (d3)) to the total content of trimethylglycine (component (d1)) and xylitol (component (d2)) [component (d3)/[component (d1) + component (d2)]] is preferably from 0.005 to 0.10, more preferably from 0.010 to 0.070, further more preferably from 0.013 to 0.050, and even more preferably from 0.013 to 0.030.

<53> The water-in-oil emulsified cosmetic according to any one of <1> to <52>, further comprising, as a component (E), an oil agent which is liquid at 25°C.

<54> The water-in-oil emulsified cosmetic according to <53>, wherein the component (E) is preferably one or more members selected from the group consisting of a silicone oil, a hydrocarbon oil, an ester oil, and a higher fatty acid; more preferably one or more members selected from the group consisting of a silicone oil and a hydrocarbon oil; and further more preferably a silicone oil (E1).

<55> The water-in-oil emulsified cosmetic according to <53> or <54>, wherein a content of the component (E) is

preferably 25 mass% or more, more preferably 27 mass% or more, and further more preferably 30 mass% or more, and is preferably 55 mass% or less, more preferably 50 mass% or less, and further more preferably 47 mass% or less.

<56> The water-in-oil emulsified cosmetic according to <53> or <54>, wherein the content of the component (E) is preferably from 25 to 55 mass%, more preferably from 27 to 50 mass%, and further more preferably from 30 to 47 mass%.

<57> The water-in-oil emulsified cosmetic according to any one of <54> to <56>, wherein a content of the component (E1) in a total amount of the component (E) in the water-in-oil emulsified cosmetic is preferably 30 mass% or more, more preferably 35 mass% or more, and further more preferably 40 mass% or more, and is preferably 85 mass% or less, more preferably 80 mass% or less, further more preferably 75 mass% or less, and even more preferably 70 mass% or less.

<58> The water-in-oil emulsified cosmetic according to any one of <54> to <56>, wherein the content of the component (E1) in the total amount of the component (E) in the water-in-oil emulsified cosmetic is preferably from 30 to 85 mass%, more preferably from 35 to 80 mass%, further more preferably from 40 to 75 mass%, and even more preferably from 40 to 70 mass%.

<59> The water-in-oil emulsified cosmetic according to any one of <53> to <58>, wherein when a silicone oil (E1) is contained as the component (E), a mass ratio of the content of the component (E1) to the content of the component (A1) [component (E1)/component (A1)] is preferably 0.80 or more, more preferably 0.85 or more, further more preferably 0.90 or more, even more preferably 0.95 or more, and even more preferably 1.0 or more, and is preferably 5.0 or less, more preferably 4.5 or less, further more preferably 3.0 or less, even more preferably 2.0 or less, even more preferably 1.7 or less, even more preferably 1.5 or less, and even more preferably 1.3 or less.

<60> The water-in-oil emulsified cosmetic according to any one of <53> to <58>, wherein when a silicone oil (E1) is contained as the component (E), the mass ratio of the content of the component (E1) to the content of the component (A1) [component (E1)/component (A1)] is preferably from 0.80 to 5.0, more preferably from 0.85 to 5.0, further more preferably from 0.90 to 4.5, even more preferably from 0.95 to 3.0, even more preferably from 0.95 to 2.0, even more preferably from 1.0 to 1.7, even more preferably from 1.0 to 1.5, and even more preferably from 1.0 to 1.3.

<61> The water-in-oil emulsified cosmetic according to any one of <1> to <60>, wherein the water-in-oil emulsified cosmetic has a yield value of preferably 0.1 Pa or more, more preferably 0.3 Pa or more, further more preferably 0.5 Pa or more, and even more preferably 0.7 Pa or more, and of preferably 30 Pa or less, more preferably 20 Pa or less, further more preferably 10 Pa or less, even more preferably 5 Pa or less, even more preferably 3 Pa or less, even more preferably 1 Pa or less, and even more preferably 0.8 Pa or less.

<62> The water-in-oil emulsified cosmetic according to any one of <1> to <60>, wherein the water-in-oil emulsified cosmetic has a yield value of preferably from 0.1 to 30 Pa, more preferably from 0.3 to 20 Pa, further more preferably from 0.3 to 10 Pa, even more preferably from 0.3 to 5 Pa, even more preferably from 0.3 to 3 Pa, even more preferably from 0.3 to 1 Pa, even more preferably from 0.5 to 1 Pa, and even more preferably from 0.7 to 0.8 Pa.

<63> The water-in-oil emulsified cosmetic according to any one of <1> to <62>, which is for use in sunscreen.

## Examples

The present invention will be described in detail below with reference to Examples and Comparative Examples; however, the present invention is not limited thereto.

(Hydrophobicity degree (%) of component (A) in accordance with methanol titration)

50 mL of ion exchange water and 0.2 g of an UV scattering agent were placed in a beaker, and methanol was added dropwise from a burette while the mixture in the beaker was stirred with a magnetic stirrer. Due to the increase in the concentration of methanol in the beaker, the UV scattering agent settled, and the mixture in the beaker became cloudy to reduce transmittance. At this point, the mass fraction of methanol in the water/methanol mixed solution at which the transmittance of the mixture after dropping methanol was 50% of the transmittance of the mixture before dropping methanol was regarded as hydrophobicity degree (%).

(Average primary particle size of component (A))

The average primary particle size of the component (A) was measured in the following manner.

In the case of a measurement sample having a shape other than a flaky or plate-like shape, a dispersion of the measurement sample prepared in advance was placed on the sample stage of a transmission electron microscope (TEM) (product name: "JEM 1400 Plus," manufactured by JEOL Ltd.) and air-dried, after which the maximum minor axes of 300 primary particles were measured in an image observed with the TEM at a magnification of 50 000 times, and the number average value thereof was taken as the average primary particle size. The maximum minor axis as mentioned herein refers

to, among the minor axes perpendicular to the major axes, a minor axis having the maximum length.

In the case of a measurement sample having a flaky or plate-like shape, the thicknesses of 300 primary particles were measured in an image observed in the same manner and under the same magnification conditions as above, and the number average value thereof was taken as the average primary particle size.

The dispersion of the measurement sample was prepared by adding 95 g of ethanol as a solvent to 5 g of the measurement sample, followed by ultrasonic dispersion.

(Surface tension of component (C))

24 mL or more of the component (C) was placed in a thoroughly cleaned glass vessel, a platinum plate was inserted as a gauge head, and the surface tension was measured using an automatic surface tensiometer (product name: "K100," manufactured by KRUSS) in an environment of 25°C. When the gauge head is inserted into the component (C), the surface tension acts along the circumference of the gauge head, the gauge head is pulled into the component (C). This pulling force was read and taken as the surface tension.

(Yield value of water-in-oil emulsified cosmetic)

Using a cone-plate rheometer (product name: "MCR300 SN073000," manufactured by Anton Paar), the shear stress of the water-in-oil emulsified cosmetic was measured under the following conditions, and the shear stress rise value was taken as the yield value.

Jig used: Cone-Plate CP50-1S
Shear rate:

$$d\gamma/dt = 0.0001 \text{ to } 1\,000 \text{ (1/s)}$$

Measurement temperature: 25°C

Examples 1 to 13 and Comparative Examples 1 to 7

Water-in-oil emulsified cosmetics were each obtained in the following manner in accordance with the formulations shown in Tables 1 and 2.

The components 4 to 6 shown in Tables 1 and 2 were heated and dissolved in the component 16 at 85°C, and the component 2 was added, after which the resulting mixture was dispersed at 85°C using a disperser (2 500 rpm) for 20 minutes to obtain a dispersed product (i).

Separately, the component 1 or 3 was added to the component 15 at 25°C, and the resulting mixture was dispersed at 25°C using a disperser (2 500 rpm) for 20 minutes to obtain a dispersed product (ii).

Then, the dispersed product (i) and the dispersed product (ii) were mixed at 85°C, and the resulting mixture was cooled to 40°C, after which the components 7 to 14 were added, followed by mixing and emulsification at 40°C using a homomixer, thereby obtaining each water-in-oil emulsified cosmetic.

The yield value of the water-in-oil emulsified cosmetic of Example 3 was 26.6 Pa.

The water-in-oil emulsified cosmetics obtained in the Examples and Comparative Examples were evaluated for the following: (1) UV protection ability, (2) wear resistance, (3) water resistance, (4) effect of improving stratum corneum through continuous application, and (5) natural appearance when applied to skin.

The results are shown in Tables 1 and 2.

(1) UV protection ability

28.5 mg of each of the water-in-oil emulsified cosmetics obtained in the Examples and Comparative Examples was uniformly applied to a PMMA plate (5 cm × 5 cm) and then dried in a cool, dark place for 15 minutes. After drying, the transmittance (%) of the absorption spectrum (wavelength: 350 nm) was measured at a total of 9 points on the PMMA plate, namely the midpoint, each vertex, and the midpoint of the side connecting each vertex, using an SPF analyzer (product name: "UV-2000S SPF Analyzer," manufactured by Labsphere), and the average transmittance of the nine points was determined. The *in vitro* SPF value was calculated from the average transmittance (%), and the UV protection ability was evaluated based on the following evaluation criteria.

(Evaluation criteria)

5: The *in vitro* SPF value was 90 or more.
4: The *in vitro* SPF value was 80 or more and less than 90.
3: The *in vitro* SPF value was 70 or more and less than 80.
2: The *in vitro* SPF value was 50 or more and less than 70.
1: The *in vitro* SPF value was less than 50.

(2) Wear resistance

200 mg of each of the water-in-oil emulsified cosmetics obtained in the Examples and Comparative Examples was uniformly applied to a quartz glass plate (10 cm × 10 cm × 0.2 cm) (manufactured by Sanriki Seisakusho Co., Ltd.) and then dried in a cool, dark place for 15 minutes. After drying, the transmittance (%) of the absorption spectrum (wavelength: 350 nm) was measured at a total of 9 points on the quartz glass plate, namely the midpoint, each vertex, and the midpoint of the side connecting each vertex, using an SPF analyzer (product name: "UV-2000S SPF Analyzer," manufactured by Labsphere), and the average transmittance of the nine points was determined. The *in vitro* SPF value before the friction test was calculated from the average transmittance (%).

Next, a friction test was performed using a friction tester (product name: "Tribomaster," manufactured by Trinity Labs) by applying a load of 20 g to a terminal with a 2 × 2 $cm^2$ cotton cloth attached, and rubbing the cosmetic coating film part on the quartz glass plate back and forth 10 times. For the cosmetic coating film on the quartz glass plate after the friction test, the *in vitro* SPF value after the friction test was calculated using the SPF analyzer in the same manner as in the calculation of the *in vitro* SPF value before the friction test.

Then, the SPF value retention rate before and after the friction test was calculated using the following formula, and the wear resistance was evaluated based on the following evaluation criteria.

SPF value retention rate (%) = (*in vitro* SPF value of cosmetic coating film after friction test)/(in *vitro* SPF value of cosmetic coating film before friction test) × 100

(Evaluation criteria)

5: The SPF value retention rate was 70% or more.
4: The SPF value retention rate was 60% or more and less than 70%.
3: The SPF value retention rate was 50% or more and less than 60%.
2: The SPF value retention rate was 30% or more and less than 50%.
1: The SPF value retention rate was less than 30%.

(3) Water resistance

28.5 mg of each of the water-in-oil emulsified cosmetics obtained in the Examples and Comparative Examples was uniformly applied to two PMMA plates (5 cm × 5 cm) and dried in a cool, dark place for 15 minutes. After drying, one of the PMMA plates coated with each water-in-oil emulsified cosmetic was slowly immersed in a water bath at 20°C and left to soak for 30 minutes while maintaining the water temperature at 20°C, after which the PMMA plate was slowly taken out and left to dry overnight.

Separately, the other PMMA plate coated with each water-in-oil emulsified cosmetic was left to dry as it was overnight.

Then, the outflow mass of the cosmetic coating film was calculated using the following formula from the weight of the PMMA plate coated with each water-in-oil emulsified cosmetic, with or without water immersion treatment, and the water resistance of the cosmetic coating film was evaluated based on the following evaluation criteria. The smaller the outflow mass is, the better the water resistance of the cosmetic coating film is, and the better its ability to prevent the UV protection components from leaking into water is.

Outflow mass (%) = [(mass of PMMA plate coated with water-in-oil emulsified cosmetic after drying) - (mass of PMMA plate coated with water-in-oil emulsified cosmetic after water immersion and drying)]/(mass of water-in-oil emulsified cosmetic applied to PMMA plate) × 100

(Evaluation criteria)

5: The outflow mass was 9% or less.
4: The outflow mass was more than 9% and less than 10%.
3: The outflow mass was 10% or more and less than 11%.
2: The outflow mass was 11% or more and less than 12%.
1: The outflow mass was 12% or more.

(4) Effect of improving stratum corneum through continuous application

32 mg of each of the water-in-oil emulsified cosmetics obtained in the Examples and Comparative Examples was continuously applied to the inside of the forearm (4 cm × 4 cm) for 3 weeks. After 3 weeks, the moisture content of the skin was measured at a temperature of 20°C and a humidity of 40 RH% using a stratum corneum moisture meter (product name: "Corneometer CM825," manufactured by Courage+Khazaka), and the effect of improving the stratum corneum through continuous application was evaluated based on the following evaluation criteria.

(Evaluation criteria)

5: The moisture content was 1.0 or more.
4: The moisture content was 0.9 or more and less than 1.0.
3: The moisture content was 0.8 or more and less than 0.9.
2: The moisture content was 0.7 or more and less than 0.8.
1: The moisture content was less than 0.7.

(5) Natural appearance when applied to skin

32 mg of each of the water-in-oil emulsified cosmetics obtained in the Examples and Comparative Examples was applied to the inside of the forearm (4 cm × 4 cm), and the whiteness of the cosmetic coating film immediately after application was evaluated by five expert panelists based on the following evaluation criteria. The evaluation scores were determined by rounding off the average values of the five expert panelists to the nearest whole number.

(Evaluation criteria)

5: Natural whiteness and not unnatural
4: Natural whiteness, but slightly unnatural
3: Neither
2: Slightly white and looks unnatural
1: Very white and looks very unnatural

[Table 1]

Table 1

| | Component | | | | Example | | | | | | | | | | | | |
|---|---|---|---|---|---|---|---|---|---|---|---|---|---|---|---|---|---|
| | No. | Type | | | 1 | 2 | 3 | 4 | 5 | 6 | 7 | 8 | 9 | 10 | 11 | 12 | 13 |
| Blending composition of water-in oil emulsified cosmetic (mass%) | 1 | Component (A) | (A1) | Dimethicone-treated zinc oxide microparticles A1-1 *1 | 5 | 19 | 25 | 19 | 19 | 19 | 19 | 19 | 19 | 19 | 19 | 19 | 19 |
| | 2 | | (A2) | Aluminum hydroxide/stearic acid-treated titanium oxide microparticles A2-1 *2 | 8 | 8 | 8 | 8 | 8 | 8 | 8 | 8 | 8 | 8 | 8 | 8 | 8 |
| | 3 | Component (A)' | Dimethicone/methicone mixture-treated zinc oxide microparticles A'-1 *3 | | | | | | | | | | | | | | |
| | 4 | Component (B) | (b1) | N-(hexadecyloxyhydroxypropyl) -N-hydroxvethylhexadecanamide *4 | 8 | 3 | 3 | 0.5 | 8 | 3 | 3 | 3 | 3 | 3 | 3 | 3 | |
| | 5 | | (b2) | Dextrin palmitate *5 | 0.8 | 0.3 | 0.3 | 0.5 | 0.8 | 0.3 | 0.3 | 0.3 | 0.3 | 0.3 | 0.3 | | 0.3 |
| | 6 | Component (B') | PEG-3 Dimethicone *6 | | 3 | 3 | 3 | 3 | 3 | 3 | 3 | 3 | 3 | 3 | 3 | 3 | 3 |
| | 7 | Component (C) | (C1) | Glycerin *7 | 1 | 1 | 1 | 1 | 1 | 1 | | 9 | 1 | 1 | 1 | 1 | 1 |
| | 8 | | | 1,3-Butylene glycol *8 | 8 | 8 | 8 | 8 | 8 | 8 | 26 | | 8 | 8 | 8 | 8 | 8 |
| | 9 | | | DPG *9 | 5 | 5 | 5 | 5 | 2 | 7 | | | 3 | 7 | 5 | 5 | 5 |
| | 10 | | (C2) | Polyoxvethylene 2-hexyldecyl ether *10 | 0.2 | 0.2 | 0.2 | 0.2 | 0.2 | 0.2 | 0.2 | 0.2 | 0.2 | 0.2 | 0.2 | 0.2 | 0.2 |
| | 11 | | Water | | 13 | 13 | 13 | 13 | 5 | 15 | 1 | 17 | 6 | 15 | 13 | 13 | 13 |
| | 12 | Component (D) | (D1) | Trimethylglycine *11 | | | | | | | | | | | 0.1 | 0.1 | 0.1 |
| | 13 | | (D2) | Xylito( *12 | | | | | | | | | | | 0.1 | 0.1 | 0.1 |
| | 14 | | (D3) | Xanthan gum *13 | | | | | | | | | | | 0.003 | 0.003 | 0.003 |
| | 15 | Component (E) | (E1) | Dimethicone *14 | 22.5 | 22.5 | 22.5 | 22.5 | 22.5 | 22.5 | 22.5 | 22.5 | 22.5 | 22.5 | 22.5 | 22.5 | 22.5 |
| | 16 | | Liquid isoparaffin *15 | | Balance | Balance | Balance | Balance | Balance | Balance | Balance | Balance | Balance | Balance | Balance | Balance | Balance |
| | Total | | | | 100 | 100 | 100 | 100 | 100 | 100 | 100 | 100 | 100 | 100 | 100 | 100 | 100 |
| Mass ratio in water-in-oil emulsified cosmetic [component (A1)/component (A)] | | | | | 0.38 | 0.70 | 0.76 | 0.70 | 0.70 | 0.70 | 0.70 | 0.70 | 0.70 | 0.70 | 0.70 | 0.70 | 0.70 |
| Surface tension of component (C) (N/m) | | | | | 0.0345 | 0.0345 | 0.0345 | 0.0345 | 0.0337 | 0.0344 | 0.0380 | 0.0716 | 0.0345 | 0.0343 | 0.0345 | 0.0345 | 0.0345 |
| Content of component (C) in water-in-oil emulsified cosmetic (mass%) | | | | | 27.2 | 27.2 | 27.2 | 27.2 | 16.2 | 31.2 | 27.2 | 26.2 | 18.2 | 31.2 | 27.2 | 27.2 | 27.2 |

(continued)

Table 1

| | Component | | Example | | | | | | | | | | | | |
|---|---|---|---|---|---|---|---|---|---|---|---|---|---|---|---|
| | No. | Type | 1 | 2 | 3 | 4 | 5 | 6 | 7 | 8 | 9 | 10 | 11 | 12 | 13 |
| Mass ratio in water-in-oil emulsified cosmetic [component (A)/component (B)] | | | 1.5 | 8.2 | 10.0 | 27.0 | 3.1 | 8.2 | 8.2 | 8.2 | 8.2 | 8.2 | 8.2 | 9.0 | 90.0 |
| Mass *ratio in water-in-oil* emulsified *cosmetic* [*component* (*A1*)/*component (A2)]* | | | *0.63* | 2.4 | 3.1 | 2.4 | 2.4 | 2.4 | 2.4 | 2.4 | 2.4 | 2.4 | 2.4 | 2.4 | 2.4 |
| Mass ratio in water-in-oil emulsified cosmetic [component (b1)/component (b2)] | | | 10.0 | 10.0 | 10.0 | 1.0 | 10.0 | 10.0 | 10.0 | 10.0 | 10.0 | 10.0 | 10.0 | - | 0.0 |
| Mass ratio in water-in-oil emulsified cosmetic [*component* (E1)/component (A1)] | | | 4.5 | 1.2 | 0.90 | 1.2 | 1.2 | 1.2 | 1.2 | 1.2 | 1.2 | 1.2 | 1.2 | 1.2 | 1.2 |
| Mass ratio in water-in-oil emulsified cosmetic [component (B')/component (B)] | | | 0.34 | 0.91 | 0.91 | 3.0 | 0.34 | 0.91 | 0.91 | 0.91 | 0.91 | 0.91 | *0.91* | 1.0 | 10.0 |
| Mass ratio in water-in-oil emulsified cosmetic [component (B')/component (b2)] | | | 3.8 | 10.0 | 10.0 | 6.0 | 3.8 | 10.0 | 10.0 | 10.0 | 10.0 | 10.0 | 10.0 | - | 10.0 |
| Evaluation | UV protection ability | | 4 | 5 | 5 | 3 | 5 | 5 | 3 | 5 | 3 | 5 | 5 | 5 | 3 |
| | Wear resistance | | 5 | 5 | 5 | 3 | 5 | 4 | 3 | 4 | 3 | 5 | 5 | 4 | 3 |
| | *Water resistance* | | 5 | 5 | 5 | 3 | 5 | 4 | 3 | 4 | 3 | 5 | 5 | 4 | 3 |
| | Effect of improving stratum corneum through continuous application | | 4 | 4 | 3 | 3 | 4 | 3 | 3 | 3 | 3 | 3 | 5 | 4 | 3 |
| | Natural appearance when apolied to skin | | 5 | 5 | 4 | 5 | 5 | 5 | 5 | 5 | 5 | 5 | 5 | 5 | 5 |

[Table 2]

Table 2

| | Component | | | | Comparative Example | | | | | | |
|---|---|---|---|---|---|---|---|---|---|---|---|
| | No. | Type | | | 1 | 2 | 3 | 4 | 5 | 6 | 7 |
| Blending composition of water-in-oil emulsified cosmetic (mass%) | 1 1 | Component (A) | (A1) | Dimethicone-treated zinc oxide microparticles A1-1 *1 | | 19 | 19 | 19 | | 2 | 4 |
| | 2 | | (A2) | Aluminum hydroxide/stearic acid-treated titanium oxide microparticles A2-1 *2 | | 8 | 8 | 6 | 8 | 8 | 8 |
| | 3 | Component (A)' | Dimethicone/methicone mixture-treated zinc oxide microparticles A'-1 *3 | | 19 | | | | 19 | | |
| | 4 | Component (B) | (b1) | N-(hexadecyloxyhydroxypropyl) -N-hydroxyethylhexadecanamide *4 | 3 | | 3 | 3 | 3 | 3 | 3 |
| | 5 | | (b2) | Dextrin palmitate *5 | 0.3 | | 0.3 | 0.3 | 0.3 | 0.3 | 0.3 |
| | 6 | Component (B') | PEG-3 Dimethicone *6 | | 3 | 6 | 3 | 3 | 3 | 3 | 3 |
| | 7 | Component (C) | (C1) | Glycerin *7 | 1 | 1 | | 1 | 1 | 1 | 1 |
| | 8 | | | 1,3-Butylene glycol *8 | 8 | 8 | | 8 | 8 | 8 | 8 |
| | 9 | | | DPG *9 | 5 | 5 | | 5 | 5 | 5 | 5 |
| | 10 | | (C2) | Polyoxyethylene 2-hexyldecyl ether *10 | 0.2 | 0.2 | | 0.2 | 0.2 | 0.2 | 0.2 |
| | 11 | | Water | | 13 | 13 | 26 | 0.1 | 13 | 13 | 13 |
| | 12 | (D) | (D1) | Trimethylglycine *11 | | | | | | | |
| | 13 | | Component (D2) | Xylitol *12 | | | | | | | |
| | 14 | | (D3) | Xanthan gum *13 | | | | | | | |
| | 15 | Component (E) | (E1) | Dimethicone *14 | 22.5 | 22.5 | 22.5 | 22.5 | 22.5 | 22.5 | 22.5 |
| | 16 | | Liquid isoparaffin *15 | | Balance | Balance | Balance | Balance | Balance | Balance | Balance |
| | Total | | | | 100 | 100 | 100 | 100 | 100 | 100 | 100 |
| Mass ratio in water-in-oil emulsified cosmetic [component (A1)/component (A)] | | | | | 0.00 | 0.70 | 0.70 | 0.76 | 0.00 | 0.20 | 0.33 |
| Surface tension of component (C) (N/m) | | | | | 0.0345 | 0.0345 | 0.0728 | 0.0369 | 0.0345 | 0.0345 | 0.0345 |

(continued)

| | Component | | Comparative Example | | | | | | |
|---|---|---|---|---|---|---|---|---|---|
| | No. | Type | 1 | 2 | 3 | 4 | 5 | 6 | 7 |
| Content of component (C) in water-in-oil emulsified cosmetic (mass%) | | | 27.2 | 27.2 | 26.0 | 14.3 | 27.2 | 27.2 | 27.2 |
| Mass ratio in water-in-oil emulsified cosmetic [component (A)/component (B)] | | | 5.8 | - | 8.2 | 7.6 | 8.2 | 3.0 | 3.6 |
| Mass ratio in water-in-oil emulsified cosmetic [component (A1)/component (A2)] | | | - | 2.4 | 2.4 | 3.2 | 0.0 | 0.3 | 0.5 |
| Mass ratio in water-in-oil emulsified cosmetic [component (b1)/component (b2)] | | | 10.0 | - | 10.0 | 10.0 | 10.0 | 10.0 | 10.0 |
| Mass ratio in water-in-oil emulsified cosmetic [component (E1)/component (A1)] | | | - | 1.2 | 1.2 | 1.2 | - | 11.3 | 5.6 |
| Mass ratio in water-in-oil emulsified cosmetic [component (B')/component (B)] | | | 0.91 | - | 0.91 | 0.91 | 0.91 | 0.91 | 0.91 |
| Mass ratio in water-in-oil emulsified cosmetic [component (B')/component (b2)] | | | 10.0 | - | 10.0 | 10.0 | 10.0 | 10.0 | 10.0 |
| Evaluation | UV protection ability | | 1 | 2 | 2 | 2 | 2 | 2 | 3 |
| | Wear resistance | | 1 | 1 | 1 | 1 | 2 | 2 | 2 |
| | Water resistance | | 1 | 1 | 1 | 1 | 2 | 2 | 2 |
| | Effect of improving stratum corneum through continuous application | | 3 | 3 | 3 | 3 | 3 | 3 | 3 |
| | Natural appearance when applied to skin | | 5 | 5 | 5 | 5 | 5 | 5 | 5 |

The details of the components used in Tables 1 and 2 are shown below.

*1: dimethicone-treated zinc oxide microparticles Al-1 (shape: spherical, hydrophobic surface treatment agent: dimethylpolysiloxane (dimethicone), hydrophobic surface treatment amount: 3 mass%, hydrophobicity: 51%, average primary particle size: 35 nm, commercial product)
*2: aluminum hydroxide/stearic acid-treated titanium oxide microparticles A2-1 (shape: needle-like, hydrophobic surface treatment agent: stearic acid, hydrophobic surface treatment amount: 8 mass%, hydrophobicity: 58%, average primary particle size: 15 nm, commercial product)
*3: dimethicone/methicone mixture-treated zinc oxide microparticles A'-1 (shape: spherical, hydrophobic surface treatment agents: dimethylpolysiloxane (dimethicone) and methylhydrogenpolysiloxane (methicone), hydrophobic surface treatment amounts: 3 mass% (amount treated with dimethicone) and 4 mass% (amount treated with methicone), hydrophobicity: 82%, average primary particle size: 25 nm, commercial product)
*4: "Sphingolipid E," manufactured by Kao Corporation
*5: "Rheopearl KL2," manufactured by Chiba Flour Milling Co., Ltd.
*6: "Silicone KF-6015," manufactured by Shin-Etsu Chemical Co., Ltd.
*7: "Concentrated Glycerin for Cosmetics," manufactured by Kao Corporation
*8: "1,3-Butylene Glycol-P," manufactured by KH Neochem Co., Ltd.
*9: manufactured by Adeka Corporation
*10: "Emulgen 1620G," manufactured by Kao Corporation
*11: "Aminocoat," manufactured by Asahi Kasei Finechem Co., Ltd.
*12: "XIVIA C," manufactured by Danisco USA Inc.
*13: "Keldent," manufactured by Sumitomo Pharma Food & Chemical Co., Ltd.
*14: "Silicone KF-96L-2CS," manufactured by Shin-Etsu Chemical Co., Ltd.
*15: "Parleam EX," manufactured by NOF Corporation

Tables 1 and 2 reveal that compared with the water-in-oil emulsified cosmetics of the Comparative Examples, the water-in-oil emulsified cosmetics of the Examples of the present invention have more excellent UV protection effects and their cosmetic coating films have more excellent wear resistance and water resistance, thereby efficiently preventing the UV protection components contained in the cosmetic coating films from leaking into water.

The water-in-oil emulsified cosmetic of Comparative Example 5 is an example in which dimethicone/methicone mixture-treated zinc oxide microparticles were used in place of the component (A1). Tables 1 and 2 reveal that compared with the water-in-oil emulsified cosmetic of Comparative Example 5, the water-in-oil emulsified cosmetic of Example 1 of the present invention has a more excellent UV protection effect and its cosmetic coating film has more excellent wear resistance and water resistance. This is considered to be because the water-in-oil emulsified cosmetic of Comparative Example 5 uses methicone as the surface treatment agent for the zinc oxide microparticles, and is thus poorly compatible with the aqueous-phase component, failing to allow the zinc oxide microparticles to be present effectively in areas where the aqueous-phase component is present when the water-in-oil emulsified cosmetic is applied, and failing to allow the zinc oxide microparticles to be distributed uniformly on the skin surface.

## Claims

**1.** A water-in-oil emulsified cosmetic comprising the following components (A), (B), and (C):

(A) an UV scattering agent having a hydrophobicity degree of 73% or less in accordance with methanol titration;
(B) an amphiphilic substance which is solid at 25°C; and
(C) an aqueous-phase component (provided that the component (B) is excluded),
the component (A) comprising dimethicone-treated zinc oxide microparticles (Al),
a mass ratio of a content of the component (A1) to a content of the component (A) [component (A1)/component (A)] being 0.38 or more,
the component (C) having a surface tension of less than 0.0728 N/m, and
a content of the component (C) being 16 mass% or more.

**2.** The water-in-oil emulsified cosmetic according to claim 1, wherein the content of the component (A) is 5 mass% or more and 40 mass% or less.

**3.** The water-in-oil emulsified cosmetic according to claim 1 or 2, wherein a mass ratio of the content of the component (A) to a content of the component (B) [component (A)/component (B)] is 1 or more and 100 or less.

**4.** The water-in-oil emulsified cosmetic according to any one of claims 1 to 3, wherein the component (C) comprises water, an alcohol (C1), and a surfactant (C2).

**5.** The water-in-oil emulsified cosmetic according to any one of claims 1 to 4, further comprising, as a component (D), one or more members selected from the group consisting of an amino acid or a salt thereof (D1), a sugar alcohol having 4 or more carbon atoms (D2), and a water-soluble polymer (D3).

**6.** The water-in-oil emulsified cosmetic according to any one of claims 1 to 5, further comprising, as a component (E), an oil agent which is liquid at 25°C, wherein the component (E) comprises a silicone oil (E1).

**7.** The water-in-oil emulsified cosmetic according to claim 6, wherein a mass ratio of a content of the component (E1) to the content of the component (A1) [component (E1)/component (A1)] is 0.80 or more and 5.0 or less.

**8.** The water-in-oil emulsified cosmetic according to any one of claims 1 to 7, wherein the component (A) further comprises hydrophobically-treated titanium oxide microparticles (A2), and a content of the component (A2) in the water-in-oil emulsified cosmetic is less than 10 mass%.

**9.** The water-in-oil emulsified cosmetic according to any one of claims 1 to 8, further comprising, as a component (B'), an amphiphilic substance which is liquid at 25°C, wherein a mass ratio of a content of the component (B') to a content of a dextrin fatty acid ester having 8 or more and 22 or less carbon atoms (b2) as the component (B) [component (B')/component (b2)] is 3 or more and 12 or less.

**10.** The water-in-oil emulsified cosmetic according to any one of claims 1 to 9, which is for use in sunscreen.

## INTERNATIONAL SEARCH REPORT

International application No.
**PCT/JP2024/006148**

### A. CLASSIFICATION OF SUBJECT MATTER

***A61K 8/27***(2006.01)i; ***A61K 8/06***(2006.01)i; ***A61K 8/29***(2006.01)i; ***A61K 8/34***(2006.01)i; ***A61K 8/44***(2006.01)i; ***A61K 8/73***(2006.01)i; ***A61Q 17/04***(2006.01)i
FI: A61K8/27; A61K8/06; A61Q17/04; A61K8/29; A61K8/34; A61K8/44; A61K8/73

According to International Patent Classification (IPC) or to both national classification and IPC

### B. FIELDS SEARCHED

Minimum documentation searched (classification system followed by classification symbols)

A61K8/27; A61K8/06; A61K8/29; A61K8/34; A61K8/44; A61K8/73; A61Q17/04

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

Mintel GNPD

### C. DOCUMENTS CONSIDERED TO BE RELEVANT

| Category* | Citation of document, with indication, where appropriate, of the relevant passages | Relevant to claim No. |
|---|---|---|
| A | JP 2014-172837 A (DHC CO.) 22 September 2014 (2014-09-22)<br>entire text | 1-10 |
| A | WO 2022/249695 A1 (SHISEIDO COMPANY, LTD.) 01 December 2022 (2022-12-01)<br>entire text | 1-10 |
| P, A | JP 2023-098745 A (KOSE CORP.) 11 July 2023 (2023-07-11)<br>entire text | 1-10 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **03 April 2024** | **14 May 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br>Telephone No. |

Form PCT/ISA/210 (second sheet) (July 2022)

**INTERNATIONAL SEARCH REPORT**
**Information on patent family members**

International application No.
**PCT/JP2024/006148**

| Patent document cited in search report | Publication date (day/month/year) | Patent family member(s) | Publication date (day/month/year) |
|---|---|---|---|
| JP 2014-172837 A | 22 September 2014 | (Family: none) | |
| WO 2022/249695 A1 | 01 December 2022 | CN 117222394 A | |
| JP 2023-098745 A | 11 July 2023 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021044975 A
- WO 2019225491 A
- JP 3187440 B
- JP 2007326902 A
- JP 2013053146 A


### Non-patent literature cited in the description


- *J. Soc. Cosm. Chem.*, 1954, vol. 5, 249-256